(19) Europäisches Patentamt
European Patent Office
Office européen des brevets

(11) EP 1 796 462 B1

(12) **EUROPÄISCHE PATENTSCHRIFT**

(45) Veröffentlichungstag und Bekanntmachung des Hinweises auf die Patenterteilung:
**25.04.2012 Patentblatt 2012/17**

(21) Anmeldenummer: **05794725.1**

(22) Anmeldetag: **21.09.2005**

(51) Int Cl.:
***A01N 43/40*** *(2006.01)* ***A01N 43/78*** *(2006.01)*

(86) Internationale Anmeldenummer:
**PCT/EP2005/010196**

(87) Internationale Veröffentlichungsnummer:
**WO 2006/037475 (13.04.2006 Gazette 2006/15)**

(54) **WIRKSTOFFE FÜR DIE SAATGUTBEHANDLUNG**
AGENTS USED FOR THE TREATMENT OF SEEDS
PRINCIPES ACTIFS POUR TRAITER DES SEMENCES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **01.10.2004 DE 102004047922**

(43) Veröffentlichungstag der Anmeldung:
**20.06.2007 Patentblatt 2007/25**

(60) Teilanmeldung:
**10175681.5 / 2 272 343**
**10175682.3 / 2 272 344**

(73) Patentinhaber: **Bayer CropScience AG**
**40789 Monheim (DE)**

(72) Erfinder:
- **GÖRGENS, Ulrich**
  **40882 Ratingen (DE)**
- **JESCHKE, Peter**
  **51467 Bergisch Gladbach (DE)**
- **LÖSEL, Peter**
  **51371 Leverkusen (DE)**
- **MALSAM, Olga**
  **51503 Rösrath (DE)**
- **NAUEN, Ralf**
  **40764 Langenfeld (DE)**
- **TIETJEN, Klaus-Günter**
  **40764 Langenfeld (DE)**
- **VELTEN, Robert**
  **40764 Langenfeld (DE)**
- **PITTA, Leonardo**
  **51371 Leverkusen (DE)**
- **ARNOLD, Christian**
  **40764 Langenfeld (DE)**
- **HEMPEL, Waltraud**
  **65835 Liederbach (DE)**
- **SANWALD, Erich**
  **24159 Kiel (DE)**

(56) Entgegenhaltungen:
**EP-A- 0 539 588**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Anmeldung betrifft die Verwendung bekannter Wirkstoffe für die Behandlung von Saatgut.

Sie betrifft weiterhin die Bekämpfung von tierischen Pflanzenschädlingen durch das Aufbringen bekannter Wirkstoffe auf den Boden.

Sie betrifft ferner die Verwendung bekannter Wirkstoffe zur Bekämpfung bestimmter Pflanzenschädlinge.

Die Wirkstoffe der Formel (I)

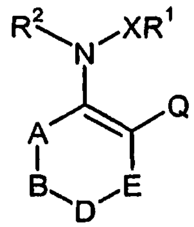

(I)

in welcher

| | |
|---|---|
| $R^1$ | für einen unsubstituierten oder substituierten 5- oder 6-gliedrigen aromatischen heterocyclischen Rest steht, der Stickstoff enthält, |
| X | für jeweils unsubstituiertes oder substituiertes Alkylen oder Alkyliden steht, |
| $R^2$ | für Wasserstoff, jeweils unsubstituiertes oder substituiertes Alkyl, Alkenyl, Cycloalkyl, Cycloalkenyl, Aryl oder für $YR^3$ steht, |
| Y | für Sauerstoff, $S(O)_1$, CO oder $CO_2$ steht, 1 für 0, 1 oder 2 steht, |
| $R^3$ | für Wasserstoff oder jeweils unsubstituiertes oder substituiertes Alkyl, Alkenyl, Alkinyl, Cycloalkyl, Cycloalkenyl oder Aryl steht, |
| A, B und D | unabhängig voneinander jeweils für ein unsubstituiertes oder substituiertes Kohlenstoffatom oder Heteroatom oder für eine Einfachbindung stehen, |
| E | für CO oder CS steht, |
| Q | für Wasserstoff oder für jeweils unsubstituiertes oder substituiertes Alkyl, Alkenyl, Alkinyl oder Aryl oder für Nitro, Halogen oder für Z-$R^4$ steht, |
| Z | für CO, $CO_2$ oder $S(O)_m$ steht, |
| m | für 0, 1 oder 2 steht und |
| $R^4$ | für jeweils unsubstituiertes oder substituiertes Alkyl, Alkenyl, Alkinyl, Cycloalkyl, Cycloalkenyl oder Aryl steht |

und ihre Herstellung sind aus EP 0 539 588 A1 bekannt.

Im einzelnen sind in dieser Publikation die folgenden Verbindungen genannt:

**Tabelle 1**

| Verbindung Nr. | 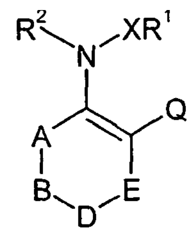 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 1 | 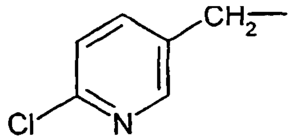 | H | $-CH_2-CH_2-CH_2-$ | 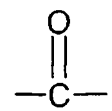 | H | [164-166] |
| 2 | 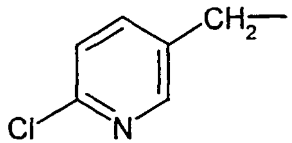 | $CH_3$ | $-CH_2-CH_2-CH_2-$ | 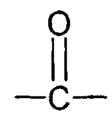 | H | [86-88] |
| 3 | 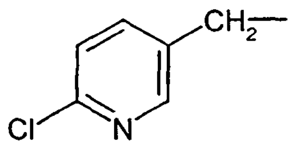 | $C_2H_5$ | $-CH_2-CH_2-CH_2-$ | 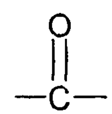 | H | $^1$H-NMR ($CDCl_3$): 5.22 |
| 4 | 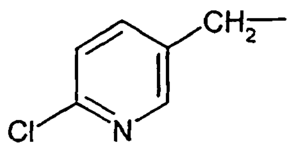 | n-$C_3H_7$ | $-CH_2-CH_2-CH_2-$ | 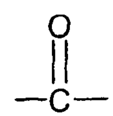 | H | $^1$H-NMR ($CDCl_3$): 5.20 |
| 5 | 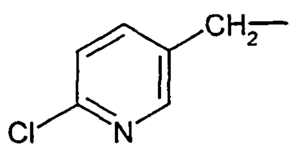 | i-$C_3H_7$ | $-CH_2-CH_2-CH_2-$ | 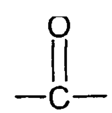 | H | |

(fortgesetzt)

| Verbindung Nr. | 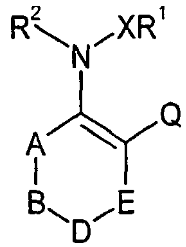 | | | | | Physikalische Eigenschaften [ ]Schmpk. ˚C LC-MS; $^1H$-NMR (Lösungsmittel) vinyl-H [ppm] |
|---|---|---|---|---|---|---|
| | $R_1$-X | $R_2$ | -A-B-D | E | Q | |
| 6 | 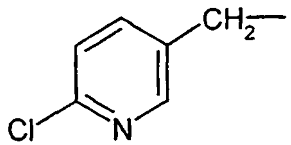 | 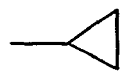 | $-CH_2-CH_2-CH_2-$ | 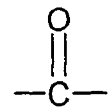 | H | |
| 7 | 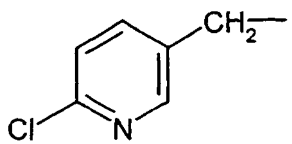 | 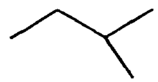 | $-CH_2-CH_2-CH_2-$ | 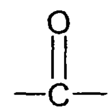 | H | $n_D^{25}$ 1.6005 |
| 8 | 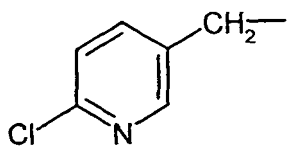 | 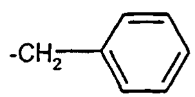 | $-CH_2-CH_2-CH_2-$ | 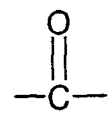 | H | |
| 9 | 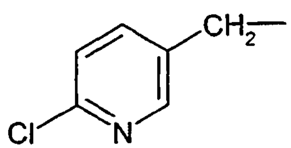 | $CH_2CH=CH_2$ | $-CH_2-CH_2-CH_2-$ | 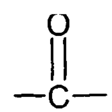 | H | $^1H$-NMR ($CDCl_3$): 5.26 |
| 10 | 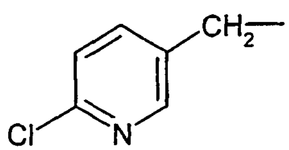 | $CH_2C=CH$ | $-CH_2-CH_2-CH_2-$ | 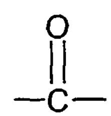 | H | $^1H$-NMR ($CDCl_3$): 5.34 |

(fortgesetzt)

| Verbindung Nr. | 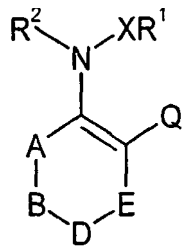 | | | | | Physikalische Eigenschaften [ ]Schmpk. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 11 | 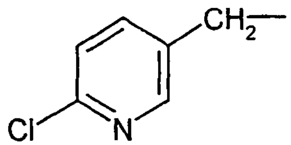 | $OCH_3$ | $-CH_2-CH_2-CH_2-$ | 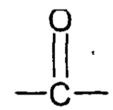 | H | [101-104] |
| 12 | 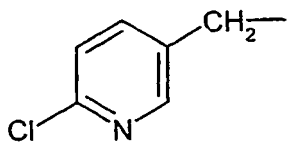 | $COCH_3$ | $-CH_2-CH_2-CH_2-$ | 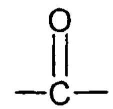 | H | |
| 13 | 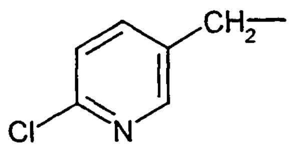 | COCF3 | $-CH_2-CH_2-CH_2-$ | 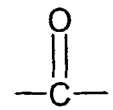 | H | $n_D^{25}$ 1.5303 |
| 14 | 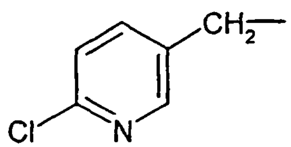 | 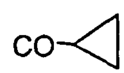 | $-CH_2-CH_2-CH_2-$ | 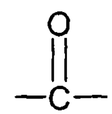 | H | |
| 15 | 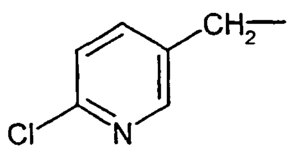 | $SO_2CH_3$ | $-CH_2-CH_2-CH_2-$ | 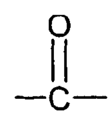 | H | |

(fortgesetzt)

| Verbindung Nr. | 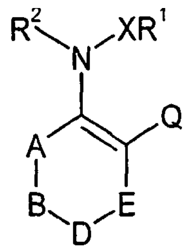 | | | | | Physikalische Eigenschaften [ ]Schmpk. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 16 | 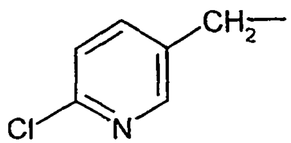 | 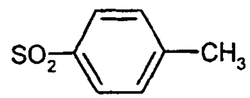 | $-CH_2-CH_2-CH_2-$ | 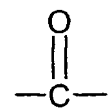 | H | |
| 17 | 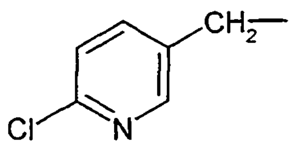 | H | $-CH_2-CH_2-CH_2-$ | 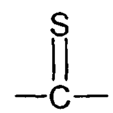 | H | [130-134] Zers. |
| 18 | 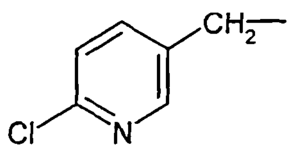 | $CH_3$ | $-CH_2-CH_2-CH_2-$ | 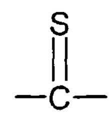 | H | |
| 19 | 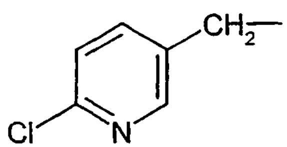 | H | $-CH_2-CH_2-CH_2-$ | 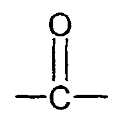 | $CH_3$ | [157-158] |
| 20 | 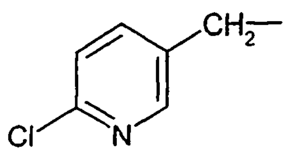 | H | $-CH_2-CH_2-CH_2-$ | 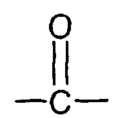 | $CH_2CH=CH_2$ | |

(fortgesetzt)

| Verbindung Nr. | 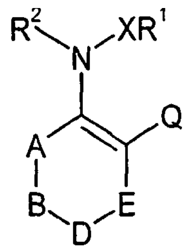 | | | | | Physikalische Eigenschaften [ ]Schmpk. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 21 | 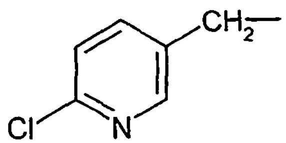 | H | $-CH_2CH_2CH_2-$ | 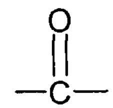 | $CH_2C{=}CH$ | |
| 22 | 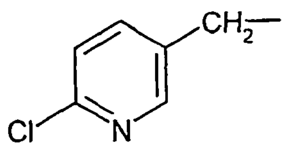 | H | $-CH_2CH_2CH_2-$ | 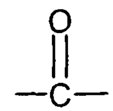 | 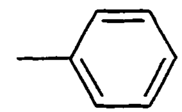 | |
| 23 | 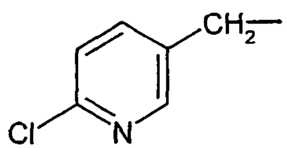 | H | $-CH_2CH_2CH_2-$ | 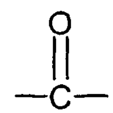 | Cl | [160-161] |
| 24 | 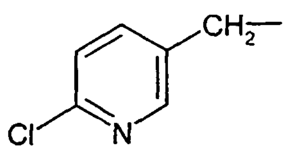 | H | $-CH_2CH_2CH_2-$ | 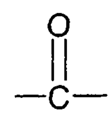 | $NO_2$ | $n_D^{25}$ 1.5908 |
| 25 | 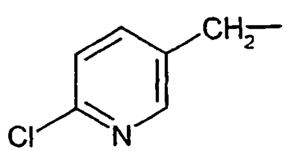 | H | $-CH_2CH_2CH_2-$ | 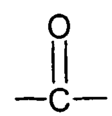 | CN | |

(fortgesetzt)

| Verbindung Nr. | 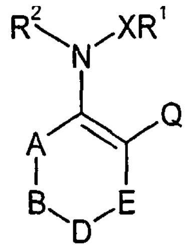 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 26 | 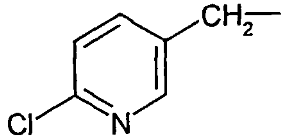 | H | $-CH_2CH_2CH_2-$ | 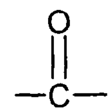 | $COCH_3$ | |
| 27 | 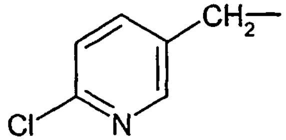 | H | $-CH_2CH_2CH_2-$ | 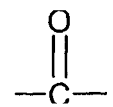 | $COCF_3$ | $n_D^{25}$ 1.5225 |
| 28 | 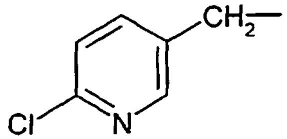 | H | $-CH_2CH_2CH_2-$ | 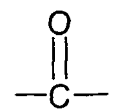 | $SO_2CH_3$ | |
| 29 | 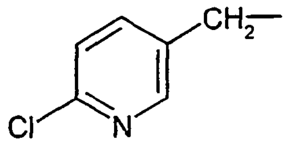 | H | 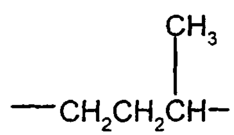 | 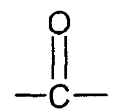 | H | [200-201] |

(fortgesetzt)

| Verbindung Nr. | 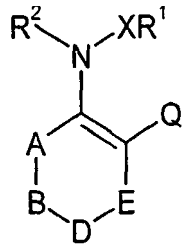 R₁-X | R2 | -A-B-D | E | Q | Physikalische Eigenschaften [ ]Schmpk. ˚C LC-MS; $^1$H-NMR (Lösungsmittel) vinyl-H [ppm] |
|---|---|---|---|---|---|---|
| 30 | 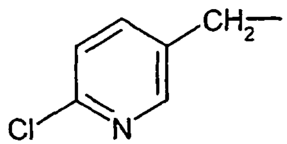 | H | 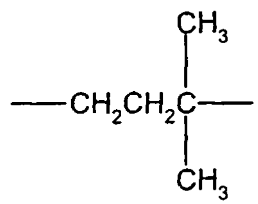 | 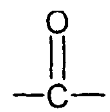 | H | [193-195] |
| 31 | 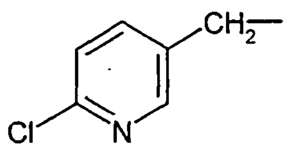 | H | 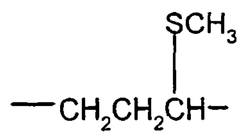 | 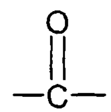 | H | [180-182] Zers. |
| 32 | 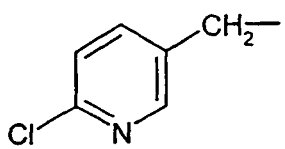 | H | 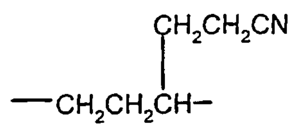 | 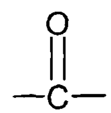 | H | [176-177] |
| 33 | 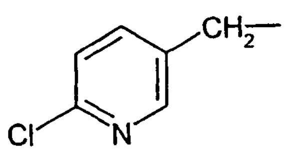 | H | 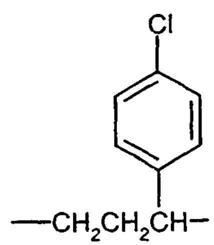 | 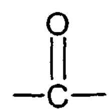 | H | [241-243] |

(fortgesetzt)

| Verbindung Nr. | 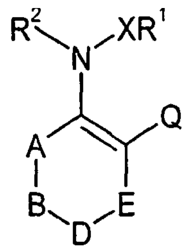 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 34 | 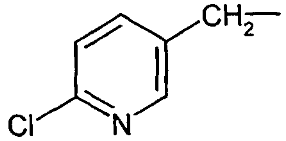 | H | 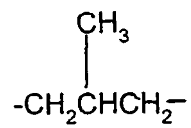 | 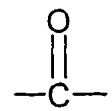 | H | [176-177] |
| 35 | 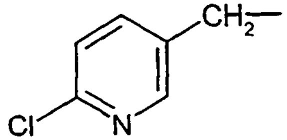 | H | 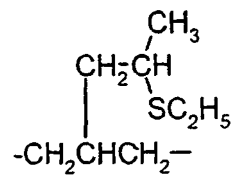 | 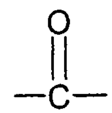 | H | [137-138] |
| 36 | 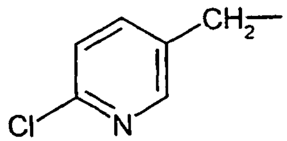 | H | 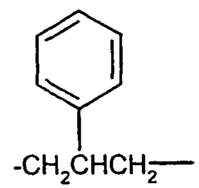 | 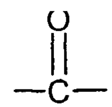 | H | [216-217] |
| 37 | 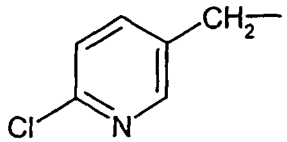 | H | 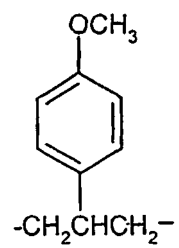 | 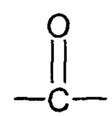 | H | [210-211] |

(fortgesetzt)

| Verbindung Nr. | 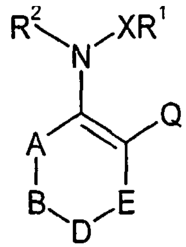 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 38 | 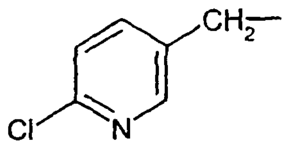 | H | 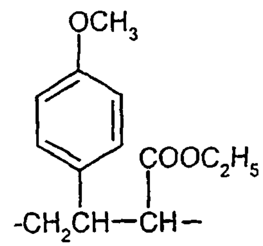 | 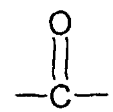 | H | [224-226] |
| 39 | 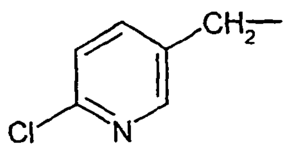 | H | 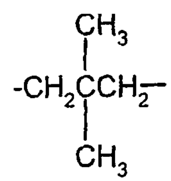 | 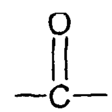 | H | [199-201] |
| 40 | 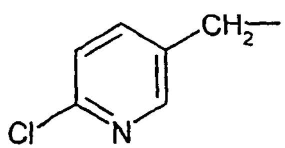 | H | 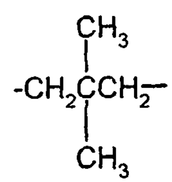 | 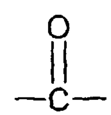 | $NO_2$ | [181-183] |

(fortgesetzt)

| Verbindung Nr. | 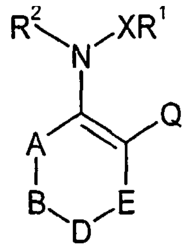 | | | | | Physikalische Eigenschaften [ ]Schmpk. ˚C LC-MS; $^{1}H$-NMR (Lösungsmittel) vinyl-H [ppm] |
|---|---|---|---|---|---|---|
| | $R_1$-X | $R_2$ | -A-B-D | E | Q | |
| 41 | 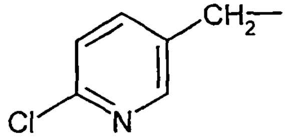 | H | 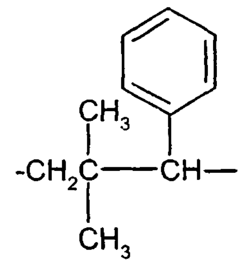 | 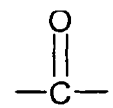 | H | [182-183] |
| 42 | 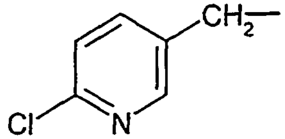 | $CH_3$ | 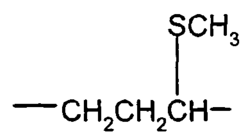 | 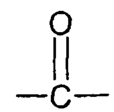 | H | [108-110] |
| 43 | 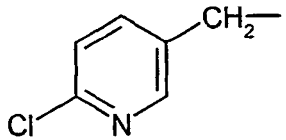 | $CH_3$ | 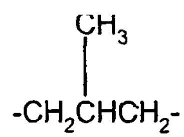 | 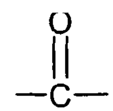 | H | $n_D^{25}$ 1.5992 |
| 44 | 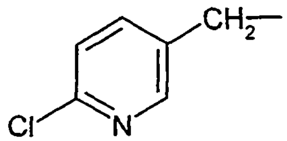 | H | 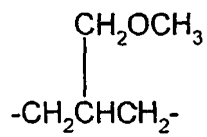 | 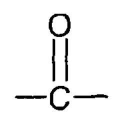 | H | [139-140] |

(fortgesetzt)

| Verbindung Nr. | 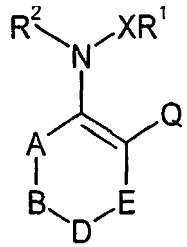 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 45 | 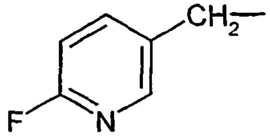 | H | $-CH_2CH_2CH_2-$ | 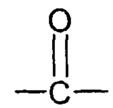 | H | |
| 46 | 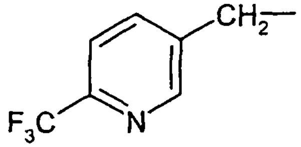 | H | $-CH_2CH_2CH_2-$ | 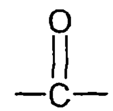 | H | |
| 47 | 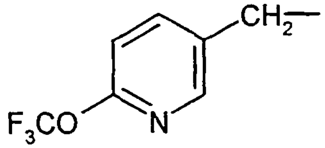 | H | $-CH_2CH_2CH_2-$ | 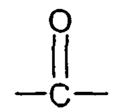 | H | |
| 48 | 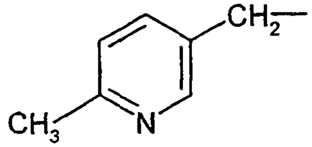 | H | $-CH_2CH_2CH_2-$ | 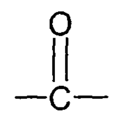 | H | |
| 49 | 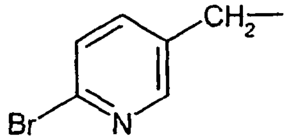 | H | $-CH_2CH_2CH_2-$ | 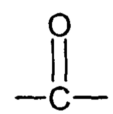 | H | |

(fortgesetzt)

| Verbindung Nr. | 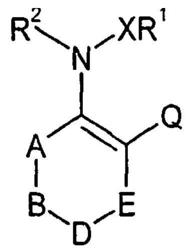 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 50 | 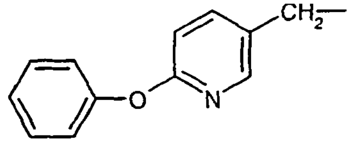 | H | $-CH_2CH_2CH_2-$ | 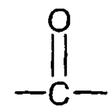 | H | |
| 51 | 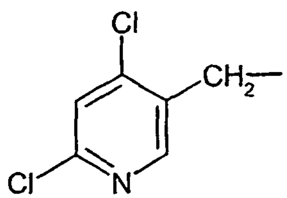 | H | $-CH_2CH_2CH_2-$ | 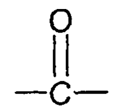 | H | |
| 52 | 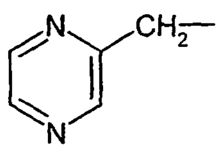 | H | $-CH_2CH_2CH_2-$ | 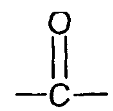 | H | |
| 53 | 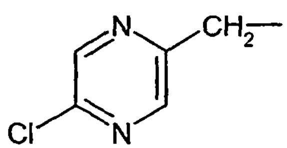 | H | $-CH_2CH_2CH_2-$ | 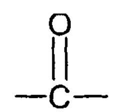 | H | |

(fortgesetzt)

| Verbindung Nr. | 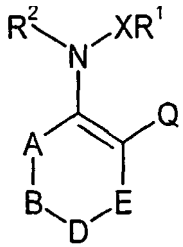 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 54 | 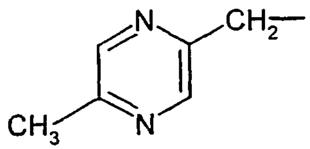 | H | $-CH_2CH_2CH_2-$ | 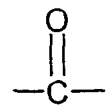 | H | |
| 55 | 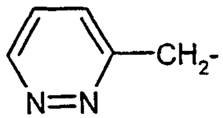 | H | $-CH_2CH_2CH_2-$ | 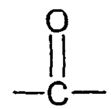 | H | |
| 56 | 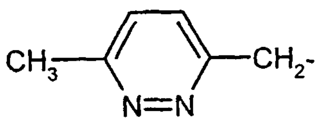 | H | $-CH_2CH_2CH_2-$ | 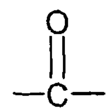 | H | |
| 57 | 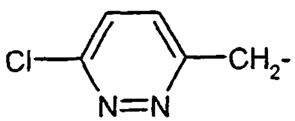 | H | $-CH_2CH_2CH_2-$ | 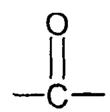 | H | |
| 58 | 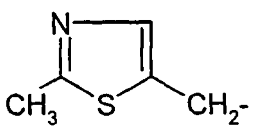 | H | $-CH_2CH_2CH_2-$ | 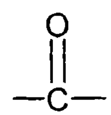 | H | |

(fortgesetzt)

| Verbindung Nr. | 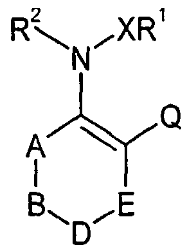 | | | | | Physikalische Eigenschaften [ ]Schmpk. ˚C LC-MS; $^1H$-NMR (Lösungsmittel) vinyl-H [ppm] |
|---|---|---|---|---|---|---|
| | $R_1$-X | $R_2$ | -A-B-D | E | Q | |
| 59 | 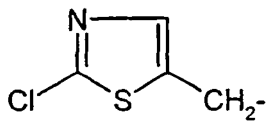 | H | $-CH_2CH_2CH_2-$ | 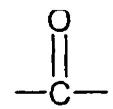 | H | [144-146] |
| 60 | 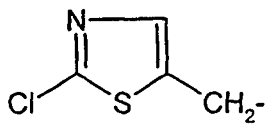 | $CH_3$ | $-CH_2CH_2CH_2-$ | 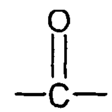 | H | $n_D^{25}$ 1.6208 |
| 61 | 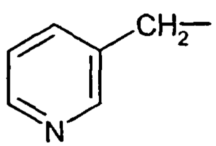 | H | $-CH_2CH_2CH_2-$ | 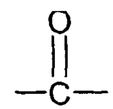 | H | [73-76] |
| 62 | 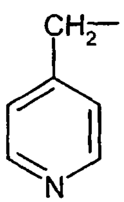 | H | $-CH_2CH_2CH_2-$ | 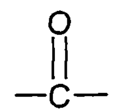 | H | |

(fortgesetzt)

| Verbindung Nr. | 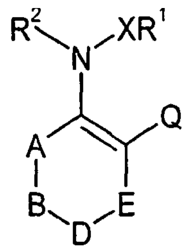 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 63 | 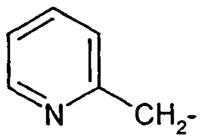 | H | $-CH_2CH_2CH_2-$ | 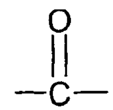 | H | |
| 64 | 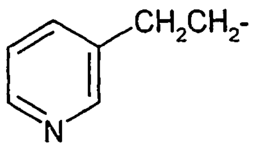 | H | $-CH_2CH_2CH_2-$ | 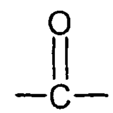 | H | |
| 65 | 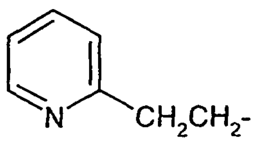 | H | $-CH_2CH_2CH_2-$ | 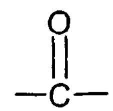 | H | |
| 66 | 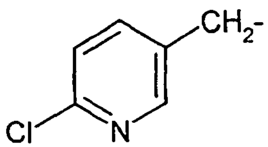 | H | $-CH_2CH_2NH-$ | 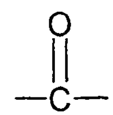 | H | [191-192] |
| 67 | 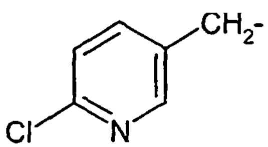 | H | 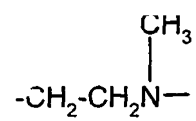 | 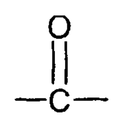 | H | |

(fortgesetzt)

| Verbindung Nr. | 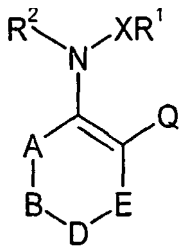 | | | | | Physikalische Eigenschaften [ ]Schmpk. ˚C LC-MS; ¹H-NMR (Lösungsmittel) vinyl-H [ppm] |
|---|---|---|---|---|---|---|
| | $R_1$-X | $R_2$ | -A-B-D | E | Q | |
| 68 | 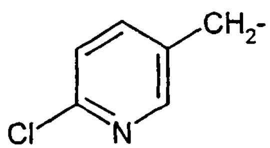 | H | 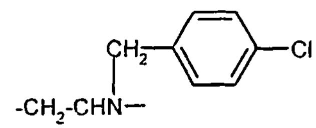 | 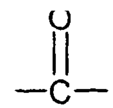 | H | [154-156] |
| 69 | 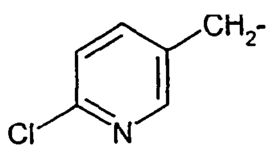 | H | 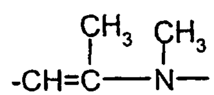 | 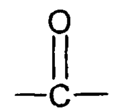 | $NO_2$ | $n_D^{25}$ 1.4972 |
| 70 | 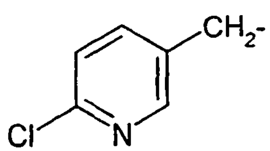 | H | 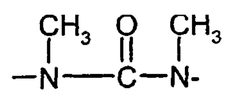 | 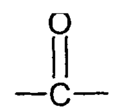 | H | [122-123] |
| 71 | 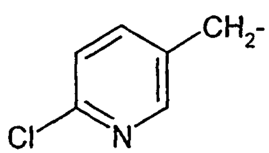 | H | 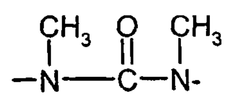 | 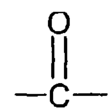 | $NO_2$ | [147-148] |
| 72 | 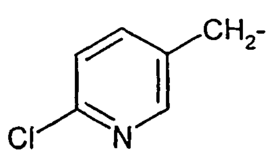 | H | 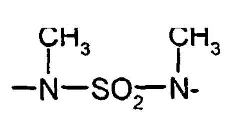 | 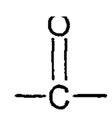 | H | [159-160] |

(fortgesetzt)

| Verbindung Nr. | 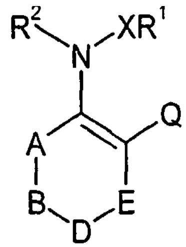 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 73 | 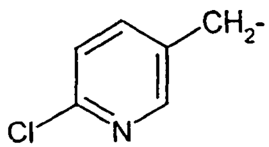 | H | 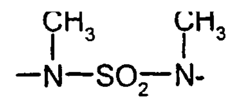 | 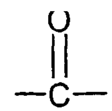 | $NO_2$ | [210-211] |
| 74 | 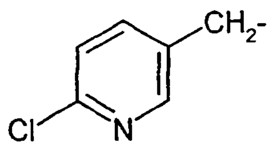 | H | $-CH_2-CH_2-O-$ | 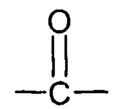 | H | [189-190] |
| 75 | 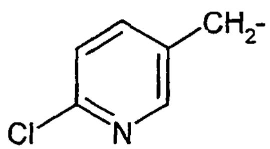 | H | 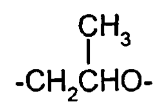 | 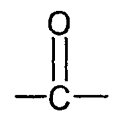 | H | [143-144] |
| 76 | 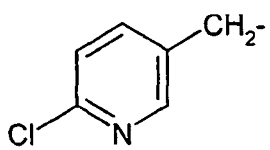 | H | 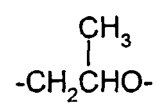 | 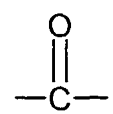 | $NO_2$ | [152-154] |
| 77 | 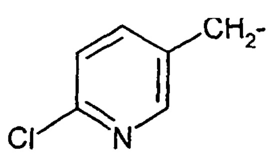 | H | 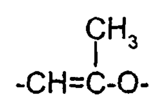 | 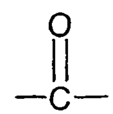 | H | [173-175] |

(fortgesetzt)

| Verbindung Nr. | 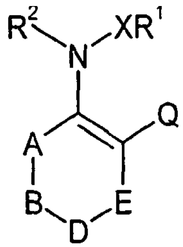 | | | | | Physikalische Eigenschaften [ ]Schmpk. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 78 | 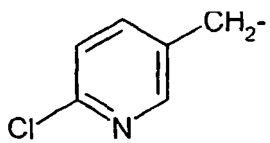 | H | 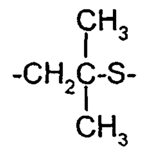 | 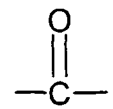 | H | [205-207] |
| 79 | 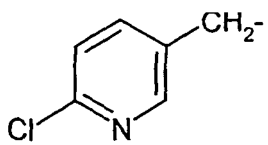 | H | 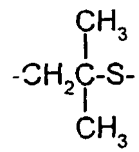 | 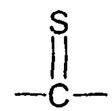 | H | [153-155] Zers. |
| 80 | 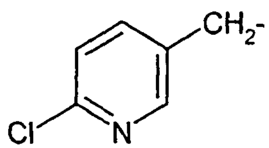 | H | $-CH_3SCH_2-$ | 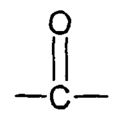 | H | [217-218] |
| 81 | 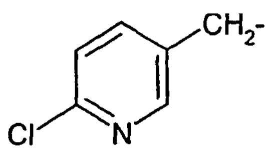 | H | 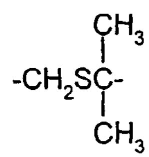 | 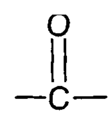 | H | [196-197] |
| 82 | 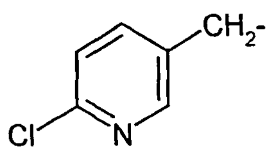 | $CH_3$ | $-CH_2CH_2O-$ | 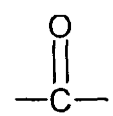 | H | [viskoses Öl] |

(fortgesetzt)

| Verbindung Nr. | 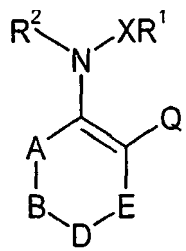 | | | | | Physikalische Eigenschaften [ ]Schmpk. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 83 | 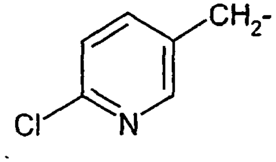 | H | $-CH_2O$ | 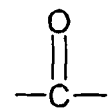 | H | [136-138] |
| 84 | 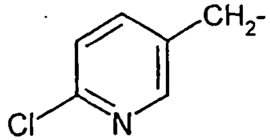 | $CH_3$ | $-CH_2O-$ | 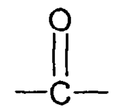 | H | [105-107] |
| 85 | 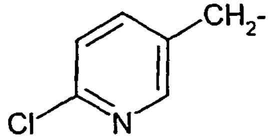 | $C_2H_5$ | $-CH_2O-$ | 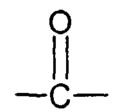 | H | [103-104] |
| 86 | 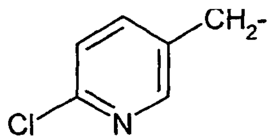 | $n-C_3H_7$ | $-CH_2O-$ | 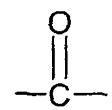 | H | [97-100] |
| 87 | 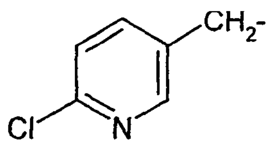 | $i-C_3H_7$ | $-CH_2O-$ | 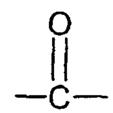 | H | $MH^+$267.1 (100); $^{1}$H-NMR ($CDCl_3$): 4.55 |

(fortgesetzt)

| Verbindung Nr. | 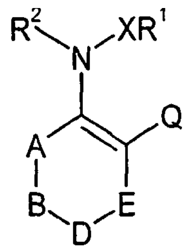 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 88 | 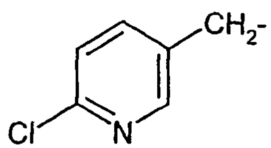 | 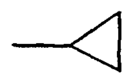 | $-CH_2O-$ | 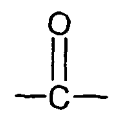 | H | [89-92] |
| 89 | 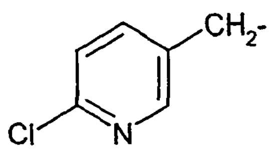 | 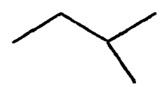 | $-CH_2O-$ | 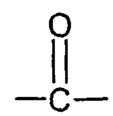 | H | $n_D^{25}$ 1.5725 |
| 90 | 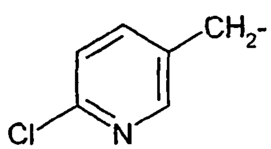 | 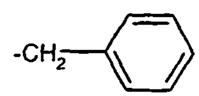 | $-CH_2O-$ | 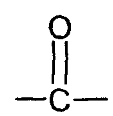 | H | 315.1 (100); $^1$H-NMR (d3-Acetonitril): 4.70 |
| 91 | 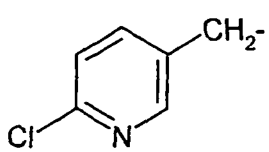 | $CH_2CH=CH_2$ | $-CH_2O-$ | 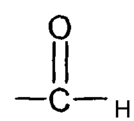 | H | [78-79] |
| 92 | 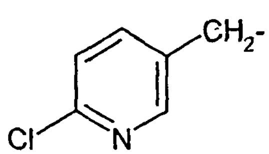 | $CH_2-C\equiv CH$ | $-CH_2O-$ | 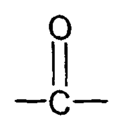 | H | $MH^+$263.0 (100); $^1$H-NMR (d3-Acetonitril): 4.90 |

(fortgesetzt)

| Verbindung Nr. | 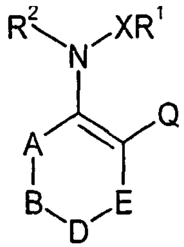 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 93 | 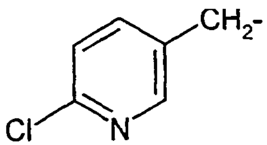 | $CONHCH_3$ | $-CH_2O-$ | 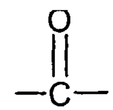 | H | [183-191] |
| 94 | 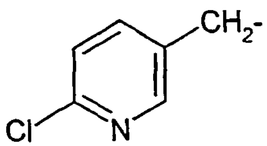 | COCH3 | $-CH_2O-$ | 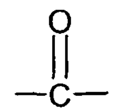 | H | $MH^+$ 267.0 (100); $^1$H-NMR ($CDCl_3$): 5.18 |
| 95 | 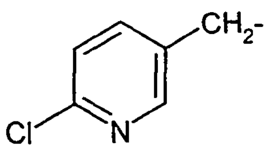 | $COCF_3$ | $-CH_2O-$ | 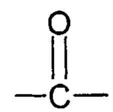 | H | |
| 96 | 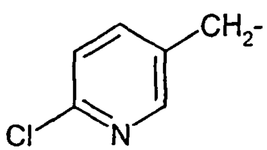 | 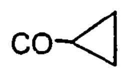 | $-CH_2O-$ | 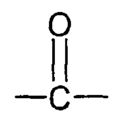 | H | [161-164] |
| 97 | 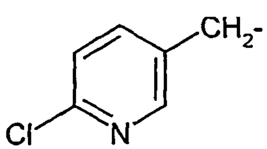 | $SO_2CH_3$ | $-CH_2O-$ | 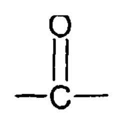 | H | |

(fortgesetzt)

| Verbindung Nr. | 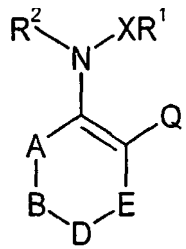 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 98 | 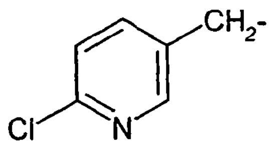 $OCH_3$ | $OCH_3$ | $-CH_2O-$ | 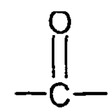 | H | [128-129] |
| 99 | 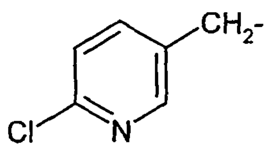 | H | $-CH_2O-$ | 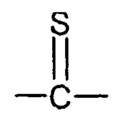 | H | |
| 100 | 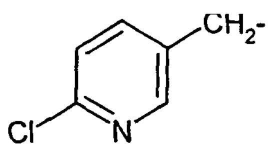 | $CH_3$ | $-CH_2O-$ | 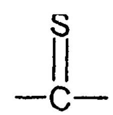 | H | |
| 101 | 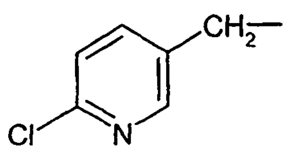 | H | $-CH_2O-$ | 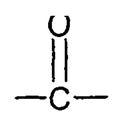 | $CH_3$ | |
| 102 | 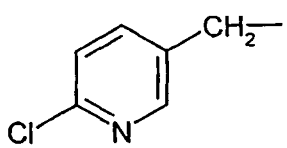 | H | $-CH_2O-$ | 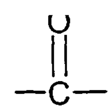 | Cl | |

(fortgesetzt)

| Verbindung Nr. | 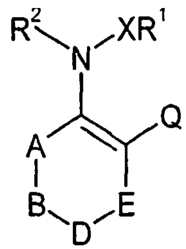 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 103 | 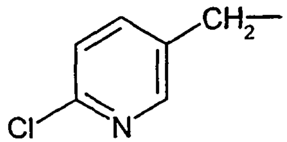 | H | $-CH_2O-$ | 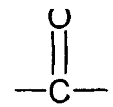 | Br | [144-145] |
| 104 | 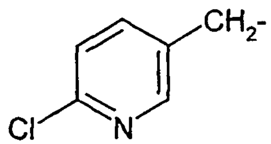 | $CH_3$ | $-CH_2O-$ | 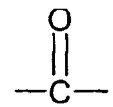 | F | [100-103] |
| 105 | 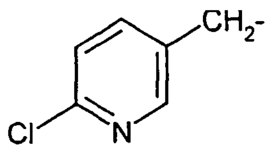 | 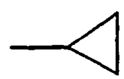 | $-CH_2O-$ | 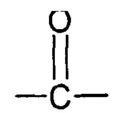 | F | [81-83] |
| 106 | 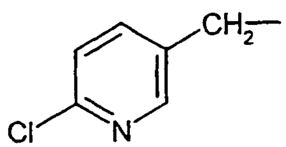 | H | $-CH_2O-$ | 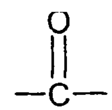 | N02 | [218-220] |
| 107 | 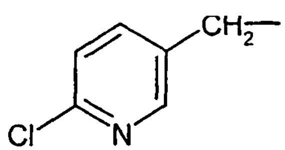 | H | $-CH_2O-$ | 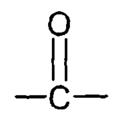 | CN | |

(fortgesetzt)

| Verbindung Nr. | 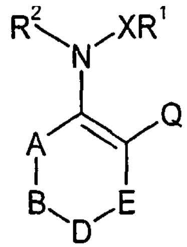 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 108 | 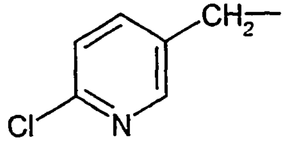 | H | $-CH_2O-$ | 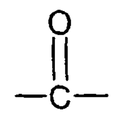 | $SO_2CH_3$ | [191-192] |
| 109 | 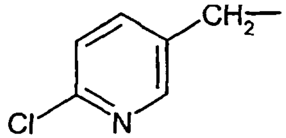 | H | $-CH_2O-$ | 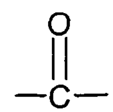 | COCH3 | |
| 110 | 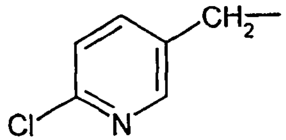 | H | $-CH_2O-$ | 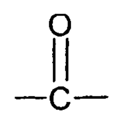 | $COCF_3$ | [173-176] Zers. |
| 111 | 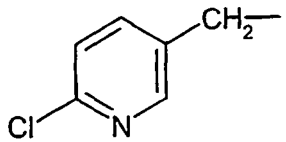 | H | $-CH_2S-$ | 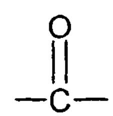 | H | [183-184] |

(fortgesetzt)

| Verbindung Nr. | 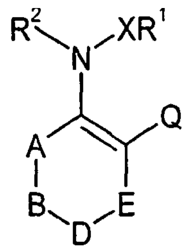 | | | | | Physikalische Eigenschaften [ ]Schmpk. ˚C LC-MS; $^1H$-NMR (Lösungsmittel) vinyl-H [ppm] |
|---|---|---|---|---|---|---|
| | $R_1$-X | $R_2$ | -A-B-D | E | Q | |
| 112 | 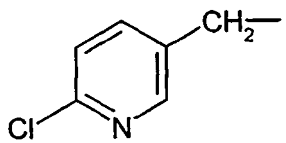 | $CH_3$ | $-CH_2S-$ | 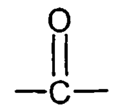 | H | [104-105] $MH^+$ 255 (100); Rt=3,10* *Rt (in min) (0,1 % HCOOH/ MeCN); vgl. WO 02085870 A |
| 113 | 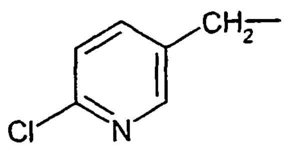 | $C_2H_5$ | $-CH_2S-$ | 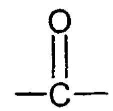 | H | $MH^+$ 269 (100); Rt = 3,61* *Rt (in min) (0,1 % HCOOH/ MeCN); vgl. WO 02085870 A |
| 114 | 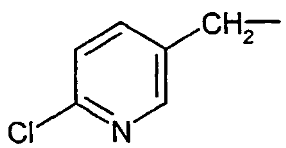 | 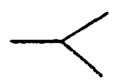 | $-CH_2S-$ | 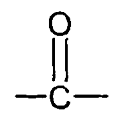 | H | |
| 115 | 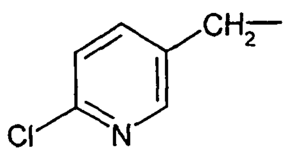 | 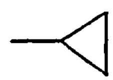 | $-CH_2S-$ | 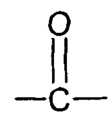 | H | |

(fortgesetzt)

| Verbindung Nr. | 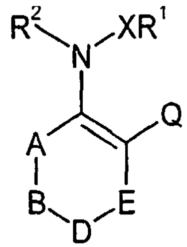 | | | | | Physikalische Eigenschaften [ ]Schmpk. °C LC-MS; $^1$H-NMR (Lösungsmittel) vinyl-H [ppm] |
|---|---|---|---|---|---|---|
| | $R_1$-X | $R_2$ | -A-B-D | E | Q | |
| 116 | 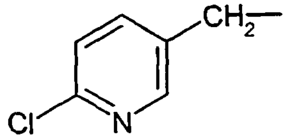 | H | 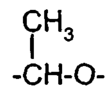 | 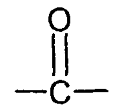 | H | [157-158] |
| 117 | 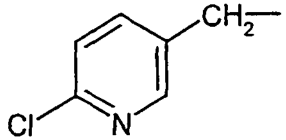 | $CH_3$ | 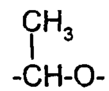 | 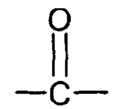 | H | $n_D^{25}$ 1.5737 |
| 118 | 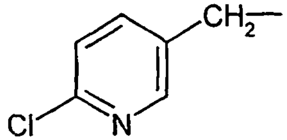 | $CH_3$ | 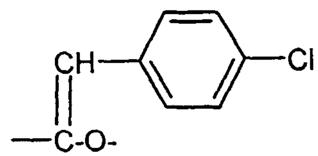 | 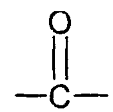 | H | [78-80] |
| 119 | 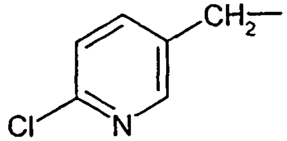 | $CH_3$ | 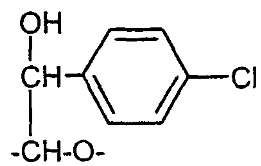 | 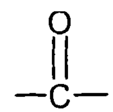 | H | [195-198] |

(fortgesetzt)

| Verbindung Nr. | 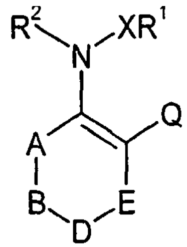 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 120 | 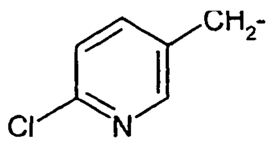 | $CH_3$ | 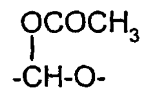 | 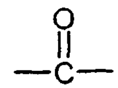 | H | [118-121] |
| 121 | 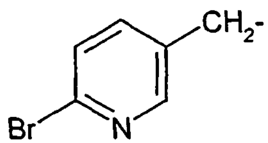 | $CH_3$ | $-CH_2O-$ | 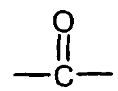 | H | $MH^+$ 283.0 (100); $^1$H-NMR (d3-Acetonitril): 4.64 |
| 122 | 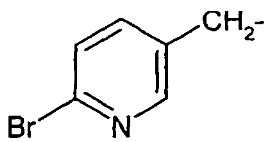 | 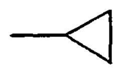 | $-CH_2O$ | 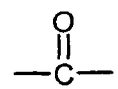 | H | |
| 123 | 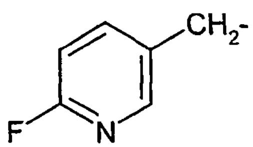 | $CH_3$ | $-CH_2O-$ | 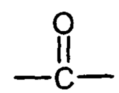 | H | $MH^+$ 223.1 (100); $^1$H-NMR (d3-Acetonitril): 4.75 |
| 124 | 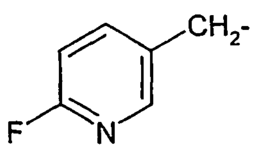 | 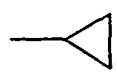 | $-CH_2O-$ | 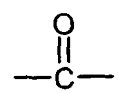 | H | $MH^+$249.1 (100); $^1$H-NMR (d3-Acetonitril): 4.75 |

(fortgesetzt)

| Verbindung Nr. | 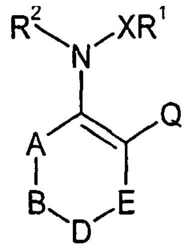 | | | | | Physikalische Eigenschaften [ ]Schmpk. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 125 | 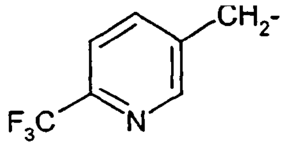 | $CH_3$ | $-CH_2O-$ | 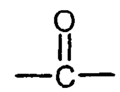 | H | $MH^+$ 273.1 (100); $^{1}$H-NMR (d3-Acetonitril): 4.64 |
| 126 | 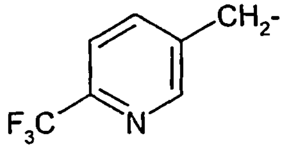 | 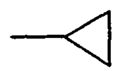 | $-CH_2O-$ | 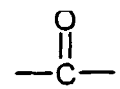 | H | $MH^+$ 299.1 (100); $^{1}$H-NMR (d3-Acetonitril): 4.72 |
| 127 | 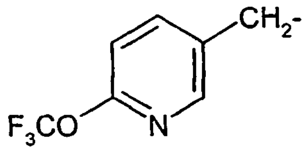 | $CH_3$ | $-CH_2O-$ | 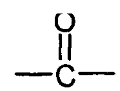 | H | |
| 128 | 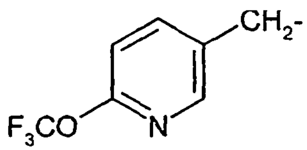 | 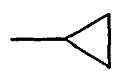 | $-CH_2O-$ | 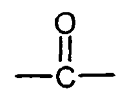 | H | |
| 129 | 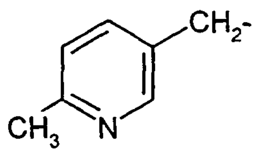 | $CH_3$ | $-CH_2O-$ | 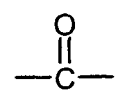 | H | |

(fortgesetzt)

| Verbindung Nr. | 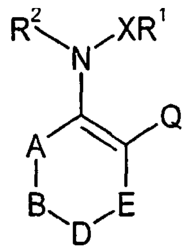 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 130 | 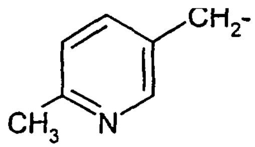 | 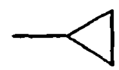 | -$CH_2$O- | 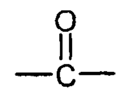 | H | |
| 131 | 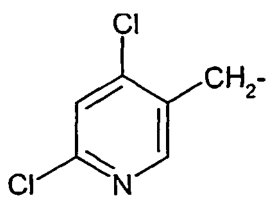 | $CH_3$ | -$CH_2$O- | 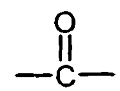 | H | |
| 132 | 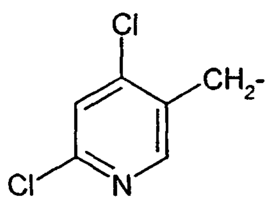 | 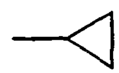 | -$CH_2$O- | 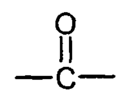 | H | |
| 133 | 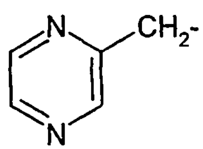 | $CH_3$ | -$CH_2$O- | 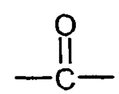 | H | |

(fortgesetzt)

| Verbindung Nr. | 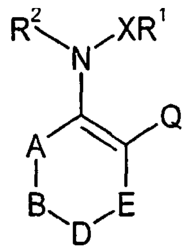 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 134 | 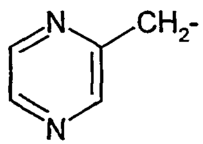 | 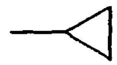 | $-CH_2O-$ | 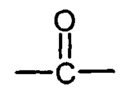 | H | |
| 135 | 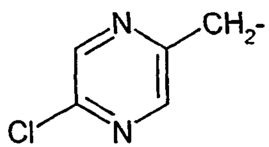 | $CH_3$ | $-CH_2O-$ | 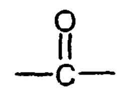 | H | |
| 136 | 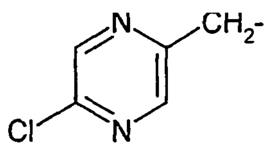 | 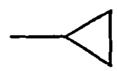 | $-CH_2O-$ | 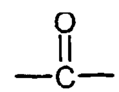 | H | |
| 137 | 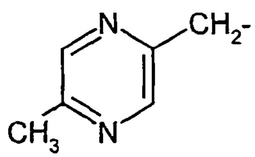 | $CH_3$ | $-CH_2O-$ | 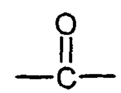 | H | |
| 138 | 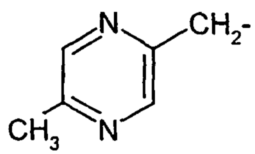 | 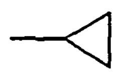 | $-CH_2O-$ | 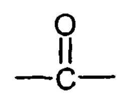 | H | |

(fortgesetzt)

| Verbindung Nr. | 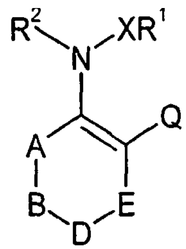 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 139 | 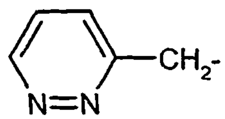 | $CH_3$ | $-CH_2O-$ | 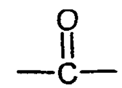 | H | |
| 140 | 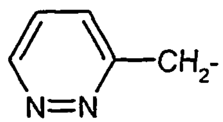 | 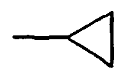 | $-CH_2O-$ | 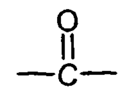 | H | |
| 141 | 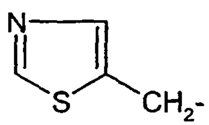 | $CH_3$ | $-CH_2O-$ | 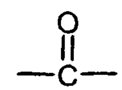 | H | |
| 142 | 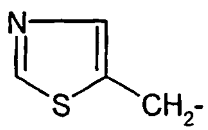 | 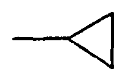 | $-CH_2O-$ | 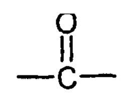 | H | |
| 143 | 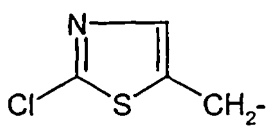 | H | $-CH_2O-$ | 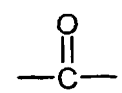 | H | [143-145] |

(fortgesetzt)

| Verbindung Nr. | 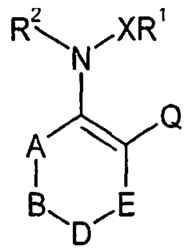 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 144 | 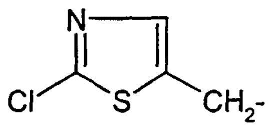 | $CH_3$ | $-CH_2O-$ | 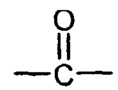 | H | $n_D^{25}$ 1.6035 |
| 145 | 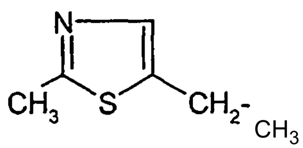 | $CH_3$ | $-CH_2O-$ | 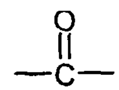 | H | |
| 146 | 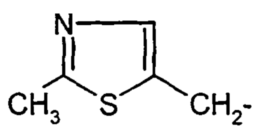 | 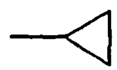 | $-CH_2O-$ | 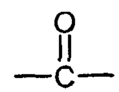 | H | |
| 147 | 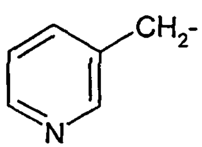 | $CH_3$ | $-CH_2O-$ | 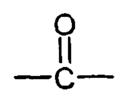 | H | $MH^+$ 205.1 (100); $^1$H-NMR (d3-Acetonitril): 4.63 |
| 148 | 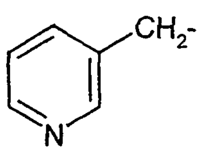 | 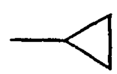 | $-CH_2O-$ | 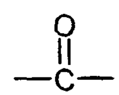 | H | |

(fortgesetzt)

| Verbindung Nr. | 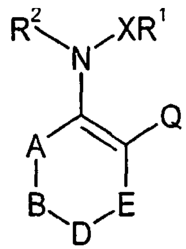 | | | | | Physikalische Eigenschaften [ ]Schmpk. ˚C LC-MS; $^1H$-NMR (Lösungsmittel) vinyl-H [ppm] |
|---|---|---|---|---|---|---|
| | $R_1$-X | $R_2$ | -A-B-D | E | Q | |
| 149 | 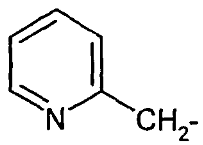 | $CH_3$ | $-CH_2O-$ | 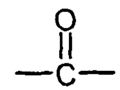 | H | |
| 150 | 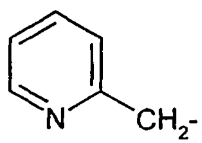 | 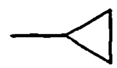 | $-CH_2O-$ | 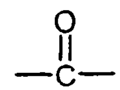 | H | |
| 151 | 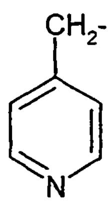 | $CH_3$ | $-CH_2O-$ | 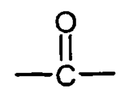 | H | |
| 152 | 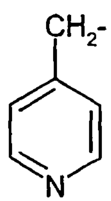 | 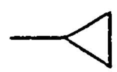 | $-CH_2O-$ | 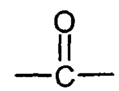 | H | |

(fortgesetzt)

| Verbindung Nr. | 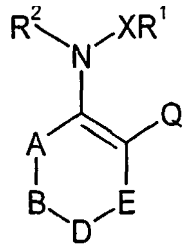 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 153 | 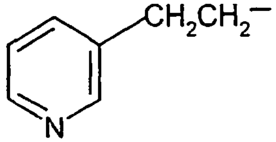 | $CH_3$ | $-CH_2O-$ | 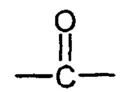 | H | |
| 154 | 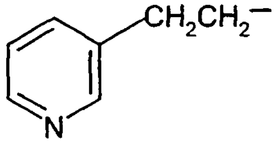 | 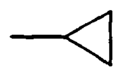 | $-CH_2O-$ | 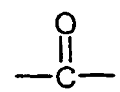 | H | |
| 155 | 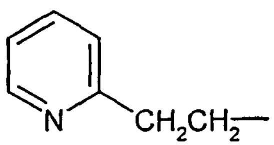 | $CH_3$ | $-CH_2O-$ | 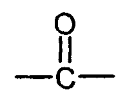 | H | |
| 156 | 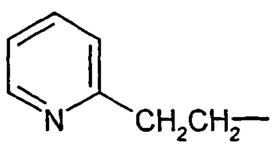 | 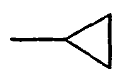 | $-CH_2O-$ | 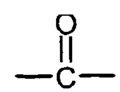 | H | |
| 157 | 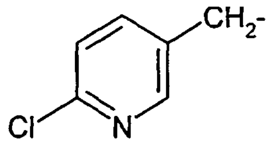 | H | $-CH_2NH-$ | 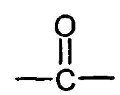 | H | [194-200] Zers. |

(fortgesetzt)

| Verbindung Nr. | 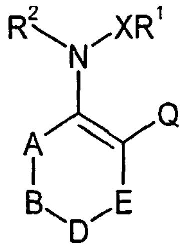 | | | | | Physikalische Eigenschaften [ ]Schmpk. ˚C LC-MS; $^1H$-NMR (Lösungsmittel) vinyl-H [ppm] |
|---|---|---|---|---|---|---|
| | $R_1$-X | $R_2$ | -A-B-D | E | Q | |
| 158 | 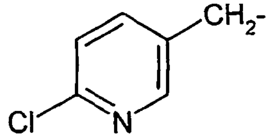 | $CH_3$ | $-CH_2NH-$ | 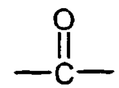 | H | [193-198] Zers. |
| 159 | 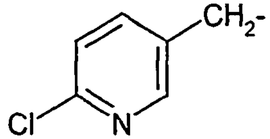 | H | 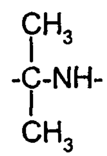 | 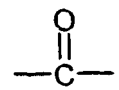 | H | [214-215] |
| 160 | 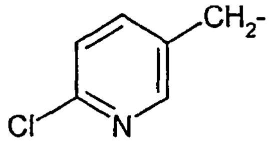 | $CH_3$ | 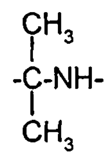 | 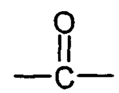 | H | [147-148] |
| 161 | 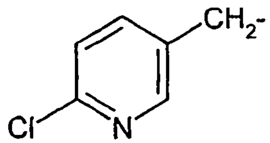 | H | 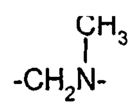 | 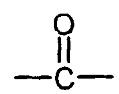 | H | [192-193] Zers. |
| 162 | 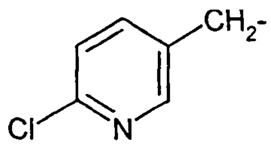 | $CH_3$ | 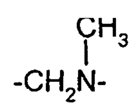 | 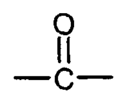 | H | [114-115] |

(fortgesetzt)

| Verbindung Nr. | 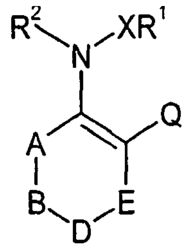 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 163 | 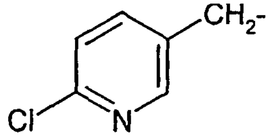 | H | 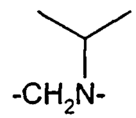 | 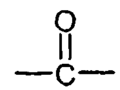 | H | [223-226] Zers. |
| 164 | 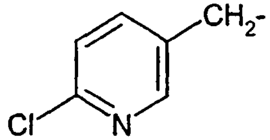 | $CH_3$ | 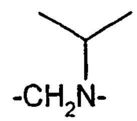 | 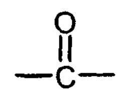 | H | [101-102] |
| 165 | 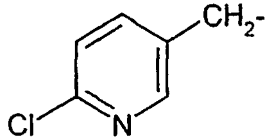 | $CH_3$ | 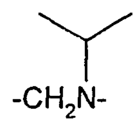 | 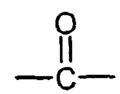 | 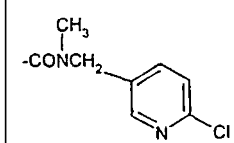 | [133-136] |
| 166 | 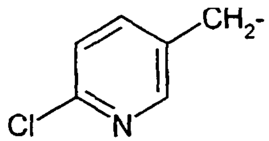 | n-$C_3H_7$ | -$CH_2$NH- | 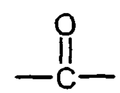 | H | |
| 167 | 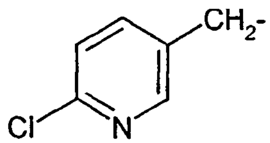 | i-$C_3H_7$ | -$CH_2$NH- | 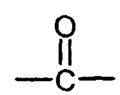 | H | |

(fortgesetzt)

| Verbindung Nr. | 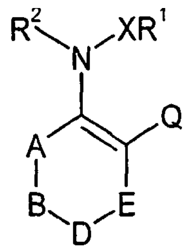 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 168 | 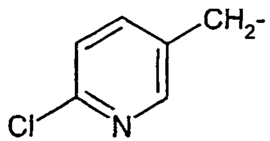 | 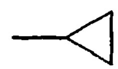 | $-CH_2NH-$ | 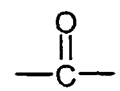 | H | $MH^+$ 264.0 (100); $^1$H-NMR (d6-DMSO): 4.77 |
| 169 | 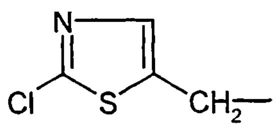 | $CH_3$ | $-CH_2NH-$ | 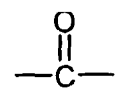 | H | $MH^+$ 244.0 (100); $^1$H-NMR (d6-DMSO): 4.80 |
| 170 | 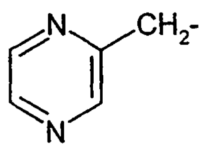 | $CH_3$ | $-CH_2NH-$ | 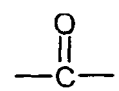 | H | |
| 171 | 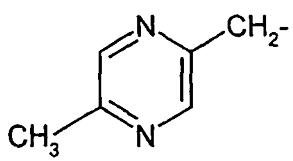 | $CH_3$ | $-CH_2NH-$ | 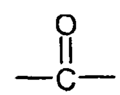 | H | |
| 172 | 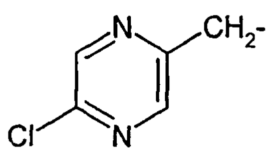 | $CH_3$ | $-CH_2NH-$ | 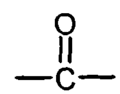 | H | |

(fortgesetzt)

| Verbindung Nr. | 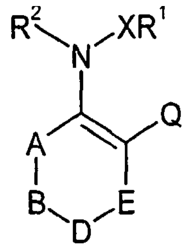 | | | | | Physikalische Eigenschaften [ ]Schmpk. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 173 | 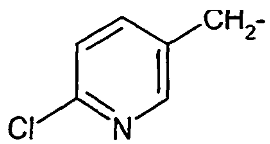 | $CH_3$ | $-CH_2NH-$ | 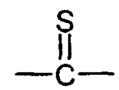 | H | |
| 174 | 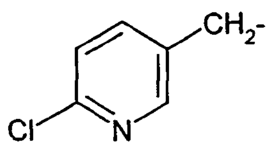 | H | $-CH_2CH_2-$ | 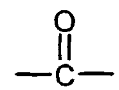 | H | [173-175] |
| 175 | 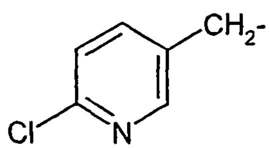 | $CH_3$ | $-CH_2CH_2-$ | 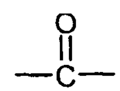 | H | $n_D^{25}$ 1.6092 |
| 176 | 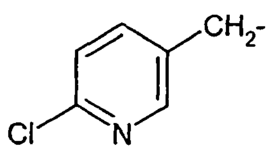 | $C_2H_5$ | $-CH_2CH_2-$ | 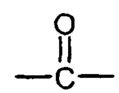 | H | |
| 177 | 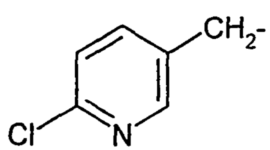 | $n-C_3H_7$ | $-CH_2$CH2- | 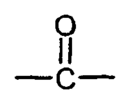 | H | |

(fortgesetzt)

| Verbindung Nr. | 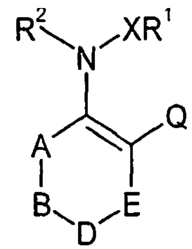 | | | | | Physikalische Eigenschaften [ ]Schmpk. ˚C LC-MS; $^{1}H$-NMR (Lösungsmittel) vinyl-H [ppm] |
|---|---|---|---|---|---|---|
| | $R_1$-X | $R_2$ | -A-B-D | E | Q | |
| 178 | 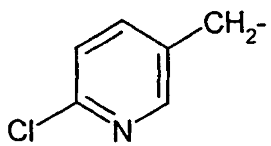 | i-$C_3H_7$ | -$CH_2$CH2- | 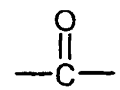 | H | |
| 179 | 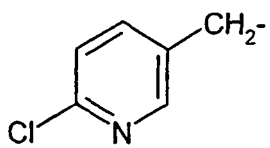 | 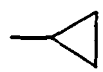 | -$CH_2CH_2$- | 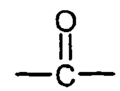 | H | $MH^+$ 263.1 (100); $^{1}H$-NMR (d6-DMSO): 5.05 |
| 180 | 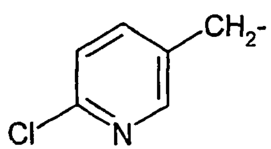 | $COCH_3$ | -$CH_2CH_2$- | 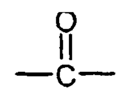 | H | |
| 181 | 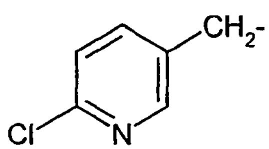 | $SO_2CH_3$ | -$CH_2CH_2$- | 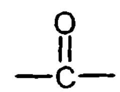 | H | |
| 182 | 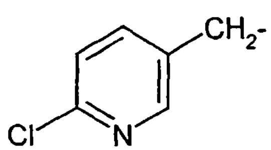 | $CH_3$ | -$CH_2CH_2$- | 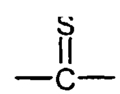 | H | |

(fortgesetzt)

| Verbindung Nr. | 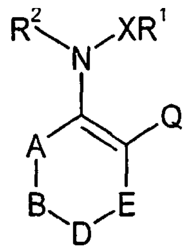 | | | | | Physikalische Eigenschaften [ ]Schmpk. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 183 | 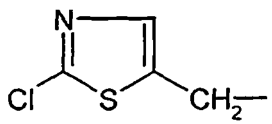 | $CH_3$ | $-CH_2CH_2-$ | 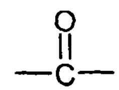 | H | |
| 184 | 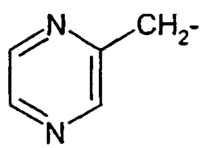 | $CH_3$ | $-CH_2CH_2-$ | 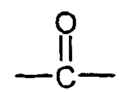 | H | |
| 185 | 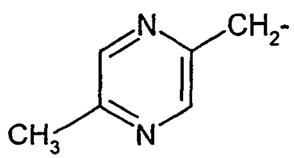 | $CH_3$ | $-CH_2CH_2-$ | 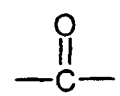 | H | |
| 186 | 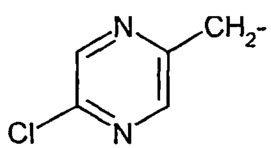 | $CH_3$ | $-CH_2CH_2-$ | 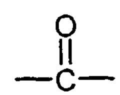 | H | |
| 187 | 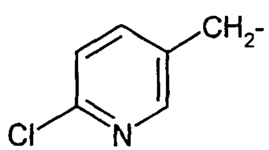 | $CH_3$ | $-CH_2CH_2-$ | 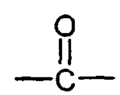 | $CH_3$ | [174-175] |

(fortgesetzt)

| Verbindung Nr. | 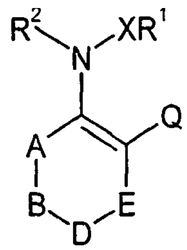 | | | | | Physikalische Eigenschaften [ ]Schmpk. ˚C LC-MS; $^1H$-NMR (Lösungsmittel) vinyl-H [ppm] |
|---|---|---|---|---|---|---|
| | $R_1$-X | $R_2$ | -A-B-D | E | Q | |
| 188 | 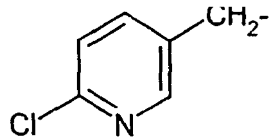 | H | $-CH_2CH_2-$ | 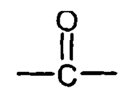 | $NO_2$ | [165-167] |

Ferner ist aus EP 0 539 588 A1 bekannt, dass die Verbindungen der Formel (I) eine gute Wirksamkeit gegen Heerwurm, Kohlmotte, Blattläuse, Zikaden und die braune Reiszikade aufweisen.

Jetzt wurde gefunden, dass die bekannten Verbindungen Nr. 84 und Nr. 88 sich hervorragend für die Behandlung von Saatgut eignen.

So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit den Verbindungen 84 und 88 behandelt wird. Die Erfindung bezieht sich ebenfalls auf die Verwendung der Verbindungen 84 und 88 zur Behandlung von Saatgut zum Schutz des Saatguts und der keimenden Pflanze vor Schädlingen. Weiterhin bezieht sich die Erfindung auf Saatgut, welches zum Schutz vor Schädlingen mit einer der Verbindungen 84 or 88 behandelt wurde.

Einer der Vorteile der vorliegenden Erfindung ist es, dass aufgrund der besonderen systemischen Eigenschaften der Verbindungen 84 und 88 die Behandlung des Saatguts mit diesen Verbindungen nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ebenso ist es als vorteilhaft anzusehen, dass die Verbindungen 84 und 88 insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit der Verbindungen 84 und 88 können bestimmte Schädlinge bereits durch die Expression des z.B. insektiziden Proteins kontrolliert werden, und es zusätzlich überraschenderweise zu einer synergistischen Wirkungsergänzung mit den Verbindungen 84 und 88 kommt, was die Effektivität des Schutzes vor Schädlingsbefall noch einmal verbessert.

Die Verbindungen 84 und 88 eignen sich zum Schutz von Saatgut jeglicher Pflanzensorte wie bereits vorstehend genannt, die in der Landwirtschaft, im Gewächshaus, in Forsten, im Gartenbau oder im Weinanbau eingesetzt wird. Insbesondere handelt es sich dabei um Saatgut von Mais, Erdnuss, Canola, Raps, Mohn, Olive, Kokosnuss, Kakao, Soja, Baumwolle, Rübe (z.B. Zuckerrübe und Futterrübe), Reis, Hirse, Weizen, Gerste, Hafer, Roggen, Sonnenblume, Zuckerrohr oder Tabak. Die Verbindungen 84 und 88 eigenen Sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit Verbindungen 84 und 88 eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie *Bacillus*, *Rhizobium*, *Pseudomonas*, *Serratia, Trichoderma, Clavibacter, Glomus* oder *Gliocladium* stammen. Die vorliegende Erfindung eignet sich besonders für die Behandlung von transgenem Saatgut, das zumindest ein heterologes Gen enthält, das aus *Bacillus sp*. stammt und dessen Genprodukt Wirksamkeit gegen Maiszünsler und/oder Maiswurzel-Bohrer zeigt. Besonders bevorzugt handelt es sich dabei um ein heterologes Gen, das aus *Bacillus thuringiensis* stammt.

Im Rahmen der vorliegenden Erfindung wird die Verbindung 84 oder 88 alleine oder in einer geeigneten Formulierung auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge der auf das Saatgut aufgebrachten Verbindung 84 oder 88 und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei

Wirkstoffen zu beachten, die in bestimmten Aufwandmengen phytotoxische Effekte zeigen können.

Die Verbindungen 84 und 88 können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186A2.

Die erfindungsgemäßen Saatgutbeizen eignen sich zur Bekämpfung von tierischen Schädlingen, vorzugsweise Arthropoden und Nematoden, insbesondere Insekten und Spinnentieren, die in der Landwirtschaft und in Forsten vorkommen. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp.

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Omithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp.

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vennicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Reticulitermes spp., Odontotermes spp.

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Cydia pomonella, Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phylloenistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.

Mit Hilfe der erfindungsgemäßen Beize sind vorzugsweise Insekten folgender Ordnungen bekämpfbar:

Bodeninsekten: Diptera (z.B. Fritfliege, Brachfliege), Coleoptera (z.B. Diabrotica, Drahtwurm), Lepidoptera (z.B. Saateule), Blattophtheroidea, Myriopoda.

Blattinsekten: Aphidina, Coleoptera, Brachycera, Lepidotera, Homoptera, Tysanoptera, Aleurodina, Cicadina, Acasi, Cossina, Heteroptera.

Überraschenderweise wurde ferner gefunden, dass die Verbindungen der Formel (I) systemische Eigenschaften besitzen und über den Boden ausgebracht eine sehr gute Wirkung gegen die oben genannten tierischen Schädlinge aufweisen.

Hier wird vorteilhaft Granulat, welches den oder die Wirkstoffe enthält, in oder auf den Boden ausgebracht. In Frage kommen beispielsweise broadcast-, Band-, Furchen- und Pflanzlochapplikation. Unter broadcast-applikation versteht man das oberflächige Ausbringen des Wirkstoffs über die gesamte zu behandelnde Fläche mit einer anschließenden

mechanischen Einarbeitung in den Boden.

Insbesondere sei die Anwendung in Pflanzkästen (Saatkästen) im Reisanbau erwähnt (nursery box treatment).

Besonders vorteilhaft ist es, die Verbindungen 84 und 88 oder ihre Salze in Wasser zu emulgieren oder zu lösen und dieses zur Bewässerung der Pflanzen zu verwenden.

In Frage kommen beispielsweise Spritzungen auf den Boden, Drenching, d.h. das Angießen der Pflanzen mit wirkstoffhaltigen Lösungen und Tröpfchenbewässerung (Drip-Irrigation), sowie die Anwendung in Hydrokulturen, insbesondere im Gemüse- und Zierpflanzenanbau.

Die Verbindungen 84 und 88 können auch über den Stamm appliziert werden, beispielsweise durch eine Stamminjektion.

Ferner wurde gefunden, dass sich die Verbindungen 84 und 88 insbesondere hervorragend zur Bekämpfung der Stubenfliege eignen.

Die Verbindung 84 oder 88 kann erfindungsgemäß bei der Bekämpfung von Schaben, also Insekten der Ordnung Blattariae, insbesondere der Familie Blattellidae, vorzugsweise der Art Blattella germanica oder der Familie Blattidae, vorzugsweise der Arten Blatta orientalis und Periplaneta americana, aber auch gegen andere Schabenarten, ganz besonders bevorzugt jedoch gegen Blattella germanica, eingesetzt werden.

Die Verbindung 84 oder 88 wirkt erfindungsgemäß auf die Schaben derart, dass die Repellentwirkung von Insektiziden, z.B. von Pyrethroiden, verringert wird.

Dieser Effekt tritt bei allen beweglichen Entwicklungsstadien (Larven, Adulte) der Schaben auf. Die para-Hydroxyphenylessigsäure und/oder ihre Mischungen mit anderen chemischen Verbindungen können somit ganz allgemein bei der Schabenbekämpfung, unabhängig von der Art der angewandten Bekämpfungsmethode eingesetzt werden. Sie kann bevorzugt bei chemischen Bekämpfungsverfahren und gegebenenfalls zusammen mit weiteren wirksamen Mitteln, wie anlockenden Ködermaterialien oder anderen Lockmitteln, synthetischen oder natürlichen Insektiziden usw. angewandt werden.

Dem Fachmann ist es anhand einfacher Überlegungen oder einfacher Untersuchungen leicht möglich, die für die jeweiligen Verwendungszwecke günstigen Mischungen sowie Anwendungsarten und Mengen zu ermitteln.

Es wurde weiterhin gefunden, dass sich die bekannten Verbindungen 84 und 88 hervorragend auch zur Bekämpfung von Schädlingen eignen, die nicht in der EP 0 539 588 genannt (Heerwurm, Kohlmotte, Blattläuse, Zikaden und die braune Reiszikade) sind.

Bevorzugt lassen sich die Verbindungen 84 und 88 zur Bekämpfung der in den Beispielen genannten Schädlinge verwenden.

Die Verbindungen 84 und 88 können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30˚C, vorzugsweise oberhalb 45˚C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet. Vorteilhaft gelangen Mineralöle mit einem Siedebereich von 170 bis 220˚C, Testbenzin mit einem Siedebereich von 170 bis 220˚C, Spindelöl mit einem Siedebereich von 250 bis 350˚C, Petroleum bzw. Aromaten vom Siedebereich von 160 bis 280˚C, Terpentinöl und dgl. zum Einsatz.

In einer bevorzugten Ausführungsform werden flüssige aliphatische Kohlenwasserstoffe mit einem Siedebereich von 180 bis 210˚C oder hochsiedende Gemische von aromatischen und aliphatischen Kohlenwasserstoffen mit einem Siedebereich von 180 bis 220˚C und/oder Spindeöl und/oder Monochlornaphthalin, vorzugsweise $\alpha$-Monochlornaphthalin, verwendet.

Die organischen schwerflüchtigen öligen oder ölartigen Lösungsmittel mit einer Verdunstungszahl über 35 und einem Flammpunkt oberhalb 30˚C, vorzugsweise oberhalb 45˚C, können teilweise durch leicht oder mittelflüchtige organisch-chemische Lösungsmittel ersetzt werden, mit der Maßgabe, dass das Lösungsmittelgemisch ebenfalls eine

Verdunstungszahl über 35 und einen Flammpunkt oberhalb 30˚C, vorzugsweise oberhalb 45˚C, aufweist und dass das Insektizid-FungizidGemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher (gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Die anwendungsfertigen Mittel können gegebenenfalls noch weitere Insektizide und gegebenenfalls noch ein oder mehrere Fungizide enthalten.

Die gute insektizide Wirkung der Verbinduhgen 84 und 88 geht aus den nachfolgenden Beispielen hervor.

<u>Beispiel F</u>

**Aphis gossypii -Test** (Bodenapplikation)

| | | |
|---|---|---|
| Lösungsmittel: | 4 | Gewichtsteile Aceton |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Erde vermischt. Die angegebene Konzentration bezieht sich auf die Wirkstoffmenge pro Volumeneinheit Boden (mg/l= ppm). Man füllt den behandelten Boden in Töpfe und bepflanzt ihn mit einer Baumwollpflanze (*Gossypium hirsutum*) bepflanzt. Nach einer Woche wird mit der Baumwollblattlaus (*Aphis gossypii*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit:

Tabelle F
pflanzenschädigende Insekten Aphis gossypii (Bodenapplikation)

| Wirkstoffe | Wirkstoffkonzentration in ppm | Wirkung in % nach $7^d$ |
|---|---|---|
| 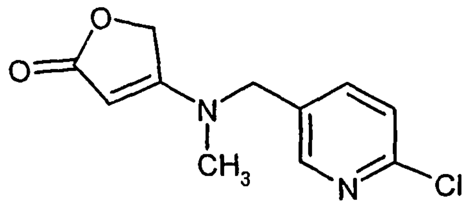 erfindungsgemäß (84) | 4 | 100 |
| 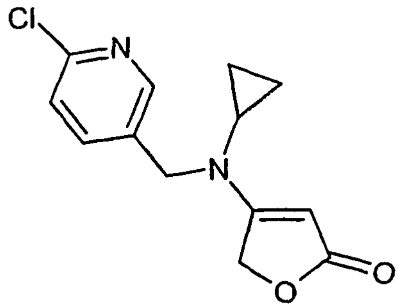 erfindungsgemäß (88) | 4 | 100 |

Beispiel G

**Myzus persicae -Test** (Bodenapplikation)

| | | |
|---|---|---|
| Lösungsmittel: | 4 | Gewichtsteile Aceton |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Erde vermischt. Die angegebene Konzentration bezieht sich auf die Wirkstoffmenge pro Volumeneinheit Boden (mg/l= ppm). Man füllt den behandelten Boden in Töpfe und bepflanzt ihn mit einer Paprikapflanze (*Capsicum annuum*) Nach einer Woche wird mit der Grünen Pfirsichblattlaus (*Myzus persicae*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit:

Tabelle G

pflanzenschädigende Insekten **Myzus persicae** (Bodenapplikation)

| Wirkstoffe | Wirkstoffkonzentration in ppm | Wirkung in % nach 7[d] |
|---|---|---|
| 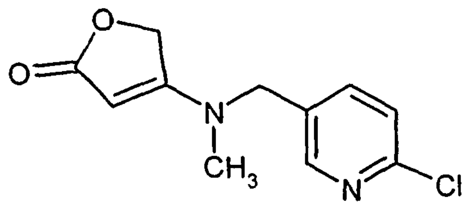 erfindungsgemäß (84) | 4 | 100 |
| 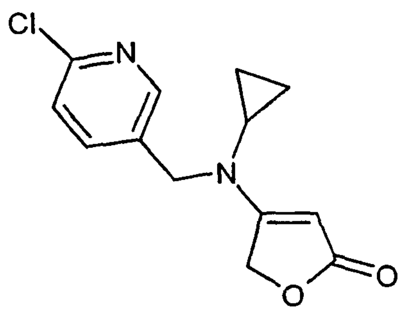 erfindungsgemäß (88) | 4 | 100 |

Beispiel H

**Diabrotica balteata - Larven -Test** (Bodenapplikation)

| | | |
|---|---|---|
| Lösungsmittel: | 4 | Gewichtsteile Aceton |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Erde vermischt. Die angegebene Konzentration bezieht sich auf die Wirkstoffmenge pro Volumeneinheit Boden (mg/l = ppm). Man füllt den behandelten Boden in Töpfe und legt je Topf 5 Maiskörner aus. 3 Tage nach Aussaat werden Larven des Maiswurzelbohrers (*Diabrotica balteata*) in den behandelten Boden gesetzt.

Nach der gewünschten Zeit wird die Abtötung in % bestimmt. Der Wirkungsgrad berechnet sich aus der Anzahl der aufgelaufenen Maispflanzen.

Bei diesem Test zeigt z. B. die folgende Verbindung der Herstellungsbeispiele gute Wirksamkeit:

Tabelle H

pflanzenschädigende Insekten

**Diabrotica balteata** (Bodenapplikation)

| Wirkstoffe | Wirkstoffkonzentration in ppm | Wirkung in % nach 4[d] |
|---|---|---|
| 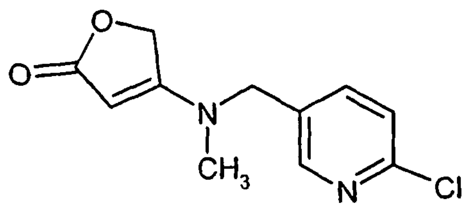 erfindungsgemäß (84) | 8 | 100 |
| 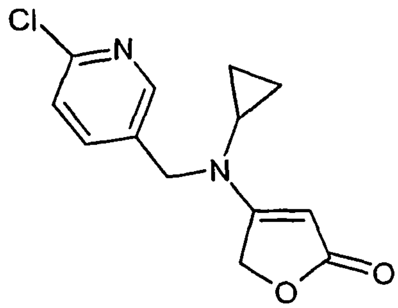 erfindungsgemäß (88) | 8 | 90 |

Beispiel I

**Aphis gossypii -Test** (Saatgutapplikation)

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Baumwollsaatgut (*Gossypium hirsufum*) wird mit der Wirkstoffzubereitung gebeizt und in Erde ausgesät. Nach ca. 2 Wochen werden die Baumwollpflanzen mit der Baumwollblattlaus (*Aphis gossypii*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit:

<u>Tabelle I</u>

pflanzenschädigende Insekten

**Aphis gossypii** (Saatgutapplikation)

| Wirkstoffe | Wirkstoffkonzentration in g ai/kg | Wirkung in % nach $7^d$ |
|---|---|---|
| 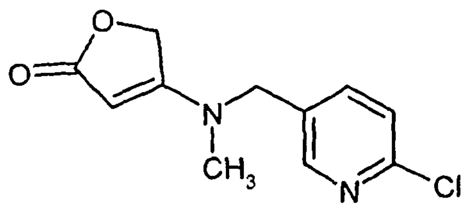 erfindungsgemäß (84) | 4 | 100 |
| 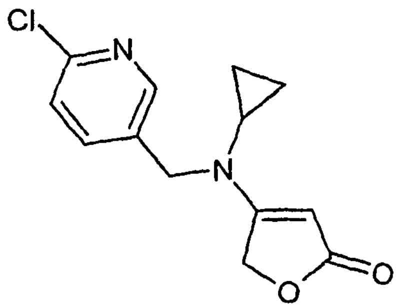 erfindungsgemäß (88) | 4 | 100 |

<u>Beispiel J</u>

**Aphis fabae -Test** (Saatgutapplikation)

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zuckerrübensaatgut (*Beta vulgaris*) wird mit der Wirkstoffzubereitung gebeizt und in Erde ausgesät. Nach ca. 4 Wochen werden die Rübenpflanzen mit der Schwarzen Bohnenblattlaus (*Aphis fabae*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindung der Herstellungsbeispiele gute Wirksamkeit:

Tabelle J

pflanzenschädigende Insekten

**Aphis fabae** (Saatgutapplikation)

| Wirkstoffe | Wirkstoffkonzentration in g ai/unit* | Wirkung in % nach 7[d] |
|---|---|---|
| 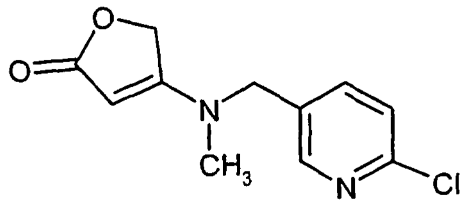 erfindungsgemäß (84) | 90 | 100 |
| * 100.000 Körner | | |

Beispiel K

**Rhopalosiphon padi -Test** (Saatgutapplikation)

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gerstensaatgut (*Hordeum vulgare*) wird mit der Wirkstoffzubereitung gebeizt und in Erde ausgesät. Nach ca. 1 Woche werden die Gerstenpflanzen mit der Haferblattlaus (*Rhopalosiphon padi*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit:

Tabelle K

pflanzenschädigende Insekten

**Rhopalosiphon padi** (Saatgutapplikation)

| Wirkstoffe | Wirkstoffkonzentration in g ai/kg | Wirkung in % nach 7[d] |
|---|---|---|
| 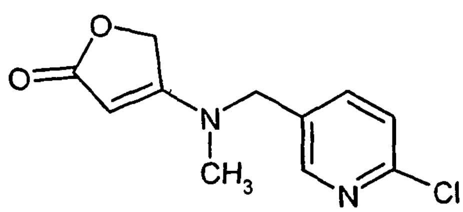 erfindungsgemäß (84) | 1,4 | 100 |

(fortgesetzt)

pflanzenschädigende Insekten

**Rhopalosiphon padi** (Saatgutapplikation)

| Wirkstoffe | | Wirkstoffkonzentration in g ai/kg | Wirkung in % nach 7[d] |
|---|---|---|---|
| 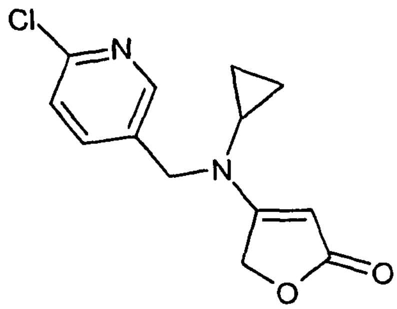 | erfindungsgemäß (88) | 1,4 | 100 |

Beispiel Z

**Bemisia tabaci -Test**

| | | |
|---|---|---|
| Lösungsmittel: | 4 | Gewichtsteile Aceton |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Erde vermischt. Die angegebene Konzentration bezieht sich auf die Wirkstoffmenge pro Volumeneinheit Boden (mg/l = ppm). Man füllt den behandelten Boden in Töpfe und bepflanzt ihn mit einer Baumwollpflanze (*Gossypium hirsutum*) bepflanzt. Nach einer Woche wird mit der Weißen Fliege (*Bemisia tabaci*) zur Eiablage infiziert.

Nach der gewünschten Zeit wird die Abtötung der Eier bzw. Larven in % bestimmt. Dabei bedeutet 100 %, dass alle Tiere abgetötet wurden; 0 % bedeutet, dass keine Tiere abgetötet wurden.

Bei einer Wirkstoffkonzentration von jeweils 4 ppm bewirkten die Verbindungen gemäß den Beispielen 84 und 88 nach 14 Tage eine Abtötung von jeweils 100 %.

Beispiel A1

**Spodoptera frugiperda -Test**

| | | |
|---|---|---|
| Lösungsmittel: | 4 | Gewichtsteile Aceton |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Erde vermischt. Die angegebene Konzentration bezieht sich auf die Wirkstoffmenge pro Volumeneinheit Boden (mg/l = ppm). Man füllt den behandelten Boden in Töpfe und bepflanzt ihn mit einer Baumwollpflanze (*Gossypium hirsutum*) bepflanzt. Nach einer Woche wird mit Raupen des Heerwurms (*Spodoptera, frugiperda*) infiziert.

Nach der gewünschten Zeit wird die Wirkung in % bestimmt. Dabei bedeutet 100 %, dass kein Fraßschaden

sichtbar ist; 0 % bedeutet, dass der Fraßschaden an den behandelten Planzen der der Kontrolle entspricht..

Bei einer Wirkstoffkonzentration von 4 ppm zeigte die Verbindung gemäß Beispiel 84 eine Wirkung von 98 % (nach 7 Tagen).

Beispiel C1

**Pulvinaria regalis -Test**

| | | |
|---|---|---|
| Lösungsmittel: | 4 | Gewichtsteile Aceton |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird an Kastanienbäume (*Castanea vesca*) angegossen. Die angegebene Konzentration bezieht sich auf die Wirkstoffmenge pro Pflanze. Nach einer definierten Zeit wird mit der Wolligen Napfschildlaus (*Pulvinaria regalis*) infiziert.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei einer Aufwandmenge von 10 mg Wirkstoff pro Pflanze bewirkte die Verbindung gemäß Beispiel 84 nach 30 Tagen eine Abtötung von 100 %.

Beispiel D1

**Aphis fabae -Test** (Saatgutapplikation)

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator urd verdünnt das Konzentrat mit Wasser auf die gewünschte Konzentration.

Zuckerrübensaatgut (*Beta vulgaris*) wird mit der Wirkstoffzubereitung gebeizt und in Erde ausgesät. Nach ca. 4 Wochen werden die Rübenpflanzen mit der Schwarzen Bohnenblattlaus (*Aphis fabae*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei einer Aufwandmenge von 90 g pro Einheit (100.000 Körner) bewirkte die Verbindung gemäß Beispiel 88 nach 7 Tage eine Abtötung von 100 %.

Beispiel L1

**Brevicoryne brassicae - Test; Freiland** (Drenchapplikation)

Zur Herstellung einer zweckmäßigen Wirkstoffzubereitung vermischt man 1 Gewichtsteil Wirkstoff mit den angegebenen Mengen Wasser auf die gewünschte Konzentration.

Kohlpflanzen (*Brassica oleracea*), die von allen Stadien der Mehligen Kohlblattlaus (*Brevicoryne brassicae*) befallen sind werden mit einer Wirkstoffzubereitung der gewünschten Konzentration angegossen.

Nach der gewünschten Zeit wird die Abtötung in % bestimmt. Dabei bedeutet 100 %, dass alle Blattläuse abgetötet wurden; 0 % bedeutet, dass keine Blattlaus abgetötet wurde.

Bei einer Aufwandmenge von 0,04 g / Pflanze bewirkten die Verbindungen gemäß den Beispielen 84 bzw. 88 nach 35 Tagen eine Abtötung von 100 bzw. 97 %.

**Patentansprüche**

1. Verwendung von Verbindungen der Formel (I)

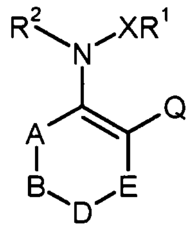

zur Behandlung von Saatgut, in denen $R^1X$, $R^2$, E, Q und die Gruppierung A-B-D folgendermaßen definiert sind

| Nr. | $R^1$-X | $R^2$ | -A-B-D | E | Q |
|---|---|---|---|---|---|
| 84 | 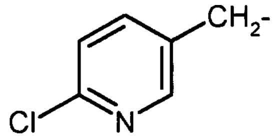 | $CH_3$ | $-CH_2O-$ | 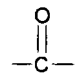 | H |
| 88 | 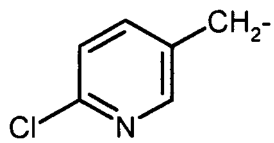 | 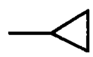 | $-CH_2O-$ | 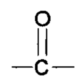 | H |

2. Die Verwendung nach Anspruch 1, wobei Saatgut von Mais, Erdnuss, Canola, Raps, Mohn, Olive, Kokosnuss, Soja, Baumwolle, Zuckerrübe, Futterübe, Reis, Hirse, Weizen, Gerste, Hafer, Roggen, Sonnenblume, Zuckerrohr oder Tabak behandelt wird.

3. Die Verwendung nach Anspruch 1, wobei transgenes Saatgut behandelt wird.

4. Saatgut, das mit mindestens einer wie in Anspruch 1 definierten Verbindung behandelt ist.

5. Das Saatgut gemäß Anspruch 4, **dadurch gekennzeichnet, dass** es Saatgut von Mais, Erdnuss, Canola, Raps, Mohn, Olive, Kokosnuss, Soja, Baumwolle, Zuckerrübe, Futterübe, Reis, Hirse, Weizen, Gerste, Hafer, Roggen, Sonnenblume, Zuckerrohr oder Tabak ist.

6. Verfahren zum Schutz von Saatgut und keimenden Pflanzen vor dem Befall von Schädlingen, indem das Saatgut mit einer Verbindung wie in Anspruch 1 definiert, behandelt wird.

7. Verfahren zur Bekämpfung von tierischen Pflanzenschädlingen, **dadurch gekennzeichnet, dass** eine Verbindung wie in Anspruch 1 definiert, auf den Boden um die Pflanzen ausgebracht wird.

8. Das Verfahren nach Anspruch 7, wobei Granulat, das eine wie in Anspruch 1 definierte Verbindung enthält in oder auf den Boden ausgebracht wird.

9. Das Verfahren nach Anspruch 7, wobei eine wie in Anspruch 1 definierte Verbindung in Wasser emulgiert oder gelöst wird und dieses zur Bewässerung der Pflanzen verwendet wird.

**Claims**

1. Use of compounds of the formula (I)

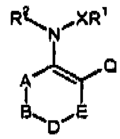

for treatment of seed, in which $R^1X$, $R^2$, E, Q and the A-B-D moiety are each defined as follows

| No. | $R^1$-X | $R^1$ | -A-B-D | E | Q |
|---|---|---|---|---|---|
| 84 | 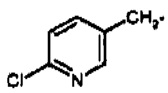 | $CH_3$ | $-CH_2O-$ | 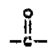 | H |
| 88 | 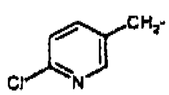 | 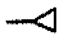 | $-CH_2O-$ | 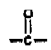 | H |

**2.** Use according to Claim 1, wherein seed of maize, peanut, canola, oilseed rape, poppy, olive, coconut, soya, cotton, sugar beet, fodder beet, rice, millet/sorghum, wheat, barley, oats, rye, sunflower, sugarcane or tobacco is treated.

**3.** Use according to Claim 1, wherein transgenic seed is treated.

**4.** Seed which has been treated with at least one compound as defined in Claim 1.

**5.** Seed according to Clam 4, **characterized in that** it is seed of maize, peanut, canola, oilseed rape, poppy, olive, coconut, soya, cotton, sugar beet, fodder beet, rice, millet/sorghum, wheat, barley, oats, rye, Sunflower, sugarcane or tobacco.

**6.** Process for protecting seed and terminating plants from infestation by pests, by treating the seed with a compound as defined in Claim 1.

**7.** Process for controlling animal plant pests, **Characterized in that** a compound as defined in Claim 1 is applied to the soil around the plants.

**8.** Process according to Claim 7, wherein granules comprising a compound as defined in Claim 1 are deployed into or onto the soil.

**9.** Process according to Claim 7, wherein a compound as defined in Claim 1 is emulsified or dissolved in water and used to water the plants.

**Revendications**

**1.** Utilisation de composés de formule (I)

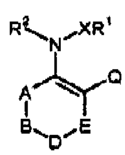

pour le traitement de semence, dans lesquels $R^1X$, $R^2$, E, Q et le groupement A-B-D sont définis comme suit

| N° | $R^1$-X | $R^2$ | -A-B-D | E | Q |
|---|---|---|---|---|---|
| 84 | 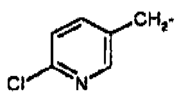 | $CH_3$ | $-CH_2O-$ | 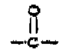 | H |
| 88 | 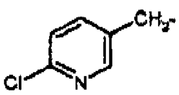 | 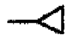 | $-CH_2O-$ | 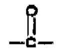 | H |

2. Utilisation selon la revendication 1, dans laquelle on traite une semence de mais, arachnide, canola, colza, pavot, olive, noix de coco, soja, coton, betterave sucrière, betterave fourragère, riz, millet, blé, orge, avoine, seigle, tournesol, canne à sucre ou tabac.

3. Utilisation selon la revendication 1, dans laquelle on traite une semence transgénique.

4. Semence, qui est traitée par au moins un composé tel que défini dans la revendication 1.

5. Semence selon la revendication 4, **caractérisée en ce qu'**il s'agit d'une semence de mais, arachide, canola, colza, pavot, olive, noix de coco, soja, coton, betterave sucrière, betterave fourragère, riz, millet, blé, orge, avoine, seigle, tournesol, canne à sucre ou tabac.

6. Procédé pour la protection de semence et de plantes en germination avant l'attaque par des ravageurs, par traitement de à semence par un composé tel que défini dans la revendication 1.

7. Procécé pour la lutte contre des ravageurs animaux de plantes, **caractérisé en ce qu'**on répand sur le sol autour des plantes un composé tel que défini dans la revendication 1.

8. Procédé selon la revendication 7, dans lequel on répand sur le sol un granulé qui contient un composé tel que défini dans la revendication 1.

9. Procécé selon la revendication 7, dans lequel on émulsionne ou dissout dans de l'eau un composé tel que défini dans la revendication 1 et on utilise cette eau pour l'irrigation des plantes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0539588 A1
- WO 02085870 A
- US 4272417 A
- US 4245432 A
- US 4808430 A
- US 5876739 A
- US 20030176428 A1
- WO 2002080675 A1
- WO 2002028186 A2
- EP 0539588 A